# EUROPEAN PATENT APPLICATION

(11) **EP 1 884 239 A1**
(43) Date of publication of application: **06.02.2008**
(21) Application number: 06118075.8
(22) Date of filing: 28.07.2006
(51) Int. Cl.: A61K 31/407, A61P 35/00

(54) **Annellated pyrrole compounds for cancer management**

(71) Applicant: MERCKLE GMBH, 89079 Ulm (DE)
(72) Inventor: Laufer, Stefan, 72076 Tübingen (DE); Guarnieri, Tiziana, 40141 Bologna (IT); Tavolari, Simona, 40033 Casalecchio di Reno (IT); Tomasi, Vittorio, 44100 Ferrara (IT); Palazzini, Ernesto, 40125 Bologna (IT); Barbanti, Miriam, 40136 Bologna (IT)
(74) Representative: Reitstötter - Kinzebach

(57) **Abstract**

The present invention relates to the use of annellated pyrrole compounds and in particular ML3000 (licofelone) 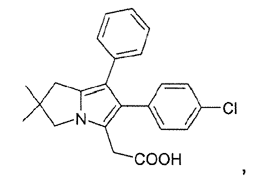 salts or derivatives thereof, for cancer management, and in general to the use of a compound of Formula (I): 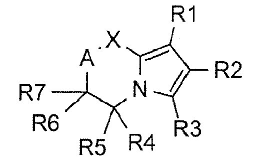 wherein the variables have the meanings given in the present description,
for the prevention and/or treatment of neoplasia, in particular neoplasia selected from the group consisting of papilloma, carcinoma, adenoma and adenocarcinoma, e. g. human airway or colorectal carcinoma, preferably fast-growing, multidrug resistant and/or COX-2 negative neoplasia.

The invention further relates to pharmaceutical compositions for the aforesaid purposes, comprising annellated pyrrole compounds and in particular ML3000 (licofelone), optical isomers, salts or derivatives thereof, as well as to a method of treating and/or preventing neoplasia and/or restoring the ability to undergo apoptosis in a cell having lost the same.

This treatment ameliorates, diminishes, actively treats, reverses or prevents any disease related to insufficient apoptosis, in particular neoplasia, and it may be combined with irradiation, heating and treatment with a cytotoxic agent for preventive and/or curative purposes.

## Description

The present invention relates to the use of annellated pyrrole compounds and in particular ML3000 (licofelone), salts or derivatives thereof, for cancer management.

### BACKGROUND OF THE INVENTION

Neoplasia is a pathological condition of regeneration-competent tissues which manifests as a deregulation of cell proliferation and differentiation and is generally perceived as uncontrolled cell proliferation. Currently, neoplastic disease is the second most frequent cause of death in the industrialized countries.

Nowadays there is no causal therapy for neoplastic disease available. Treatment basically consists of surgical removal or chemical (cytostatics) or radiological destruction of the aberrant cells and can severely impair a patient's quality of life. Thus there is strong interest in advances in therapy, in particular in achieving higher target cell selectivity of antineoplastic treatment, as well as in tumour prevention by avoidance or inactivation of risk factors.

Even with ideal selectivity of treatment, the violent destruction of neoplastic cells will lead to "unclean" necrotic cell death which results in the leakage of cell contents and fragments into the extracellular environment, thus promoting inflammation and other undesirable side effects. Attempts are therefore made to induce the less noxious programmed cell death (apoptosis) in neoplastic cells. Apoptosis is, in fact, a physiological process in which a cell undergoes a series of well-defined steps, eventually leading to orderly self-destruction without the release of toxins, proinflammatory substances etc. into the surrounding tissue. The inability of cells to self-destroy by apoptosis is considered an important factor for the development and progression of neoplasia, particularly neoplastic diseases.

In fact, in contrast to normal cells, which often react to irreparable damage by apoptosis, so to minimize danger to the organism as a whole, in neoplastic cells, the readiness to undergo apoptosis is generally reduced and they preferentially die by necrosis, thereby increasing the side effects of therapy. Promoting readiness for apoptosis in neoplastic cells could, therefore, either directly lead to their less injurious removal or increase their sensitivity to conventional treatments, which would allow to both reduce dosage (and thus systemic side effects) and secondary effects caused by necrotic cell death.

Apart from environmental factors such as ionizing radiation or chemical agents, genetic factors are known to influence a subject's propensity to develop neoplastic disease. A number of genetic factors have been characterized which interfere with natural detoxification and repair systems, thus increasing sensitivity to environmental influences up to the point where ubiquitous factors are sufficient to induce tumours, which tend to recur even after successful surgical, pharmacological or radiological intervention. This has particular significance for especially challenged tissues such as the colon epithelium, which is subject to permanent abrasion necessitating continuous replacement growth, while at the same time being exposed to microbial and chemical influences.

Among the best-known cases of genetic predisposition for neoplastic disease there are particular forms of breast cancer and retinoblastoma as well as xeroderma pigmentosum ("XP") and familial adenomatous polyposis ("FAP"). FAP is a disease with an incidence of about 1 in 10'000 and known to be caused by a mutation either in the tumour suppressor gene APC (on chromosome 5: 5q21-q22) or the DNA repair enzyme gene MYH glycosylase (on chromosome 1: p34.3-p32.1), characterized by formation of hundreds of independent polyps in the colon epithelium, beginning in late teenage and often accompanied by dermal, ocular and osteal manifestations. In 95% of all cases, these polyps later develop into carcinomas, necessitating aggressive treatment.

Nonsteroidal antiphlogistics ("NSAIDs"), such as acetylsalicylic acid ("ASA"), diclofenac, indomethacin, ibuprofen and naproxen, are widely used in the clinic for the treatment of inflammatory conditions. From a pharmacological point of view they act as inhibitors of cyclooxygenase ("COX"), an enzyme which catalyzes the formation of a number of potent local proinflammatory mediators from arachidonic acid ("AA", 5,8,11,14-eicosatetraenoic acid), well known to the skilled artisan.

Of the two COX isoforms which have been characterized, COX-1 has been described as a housekeeping enzyme and COX-2 as involved in inflammation, reproduction and carcinogenesis (Ristimaki A (2004), Cyclooxygenase 2: from inflammation to carcinogenesis. Novartis Found Symp. 2004; 256:215-226; Bernardeau-Mozer M, In-hibiteurs de la cyclo-oxygénase 2 et cancer colorectal. Bull Cancer. 2004 May 1; 91 Suppl 2: S89-98). NSAIDs have been shown to inhibit polyp formation (Giardiello F et al., N Eng J Med 1993;328:1313-6; Steinbach G et al., J Eng J Med 2000;342:1946-52), but not tumourigenic progression in FAP (Niv Y (1994), Adenocarcinoma in the rectal segment in familial polyposis is not prevented by sulindac therapy. Gastroenterology 1994 Sep; 107(3):854-857; Lynch HT (1995), Rectal cancer after prolonged sulindac chemoprevention - a case report. Cancer 1995 Feb 15; 75(4):936-8).

Another enzyme known to be involved in arachidonic acid metabolism is 5-lipoxygenase ("5-LOX"), which catalyzes leukotriene ("LT") production and thereby contributes to inflammation, cellular proliferation and atherogenesis (Steinhilber D (1999), 5-Lipoxygenase: a target for antiinflammatory drugs revisited. Curr Med Chem. 1999 Jan; 6(1):71-85; Vila L (2004), Medicinal Research Review 24: 399-424).

Pyrrolizines, which pharmacologically act in a fashion similar to NSAIDs, are known from numerous publications. For instance, antiphlogistically active pyrrolizines are described in Arch. Pharm. 319, 65-69 (1986); 319, 231-234 (1986); 318, 661-663 (1985); 318, 663-664 (1985); 319, 500-505 (1986); 319, 749-755 (1986); 327, 509-514 (1994); 330, 307-312 (1997) as well as in J. Med. Chem. 1987, 30, 820-823 and 1994, 37, 1894-1897.

Further pyrrolizines can be taken from US 5,260,451 (corresponding to EP 0397175) as well as from WO 95/32970; WO 95/32971; and WO 95/32972. These compounds are represented by the structural formulae 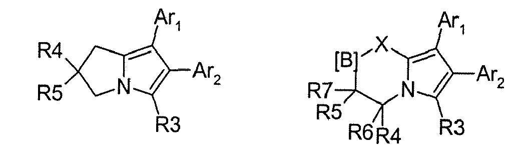 and share an annellated diarylpyrrol moiety as well as a third acidic residue R3. The compounds are characterized by a high lipophilicity, good bioavailability and half-lives in the medium range, s. Drugs of the Future, 1995, 20 (10):1007-1009.

Further pyrrolizines of similar constitution are described in DE 198 45 446.6 and WO 01/05792. Moreover, alkylsulfinylbenzoyl and alkylsulfonylbenzoyl substituted pyrrolizines, according to US 4,232,038, are said to have anti-inflammatory, analgetic and antipyretic properties. According to DE 196 24 290.8 and DE 196 24 289.4 certain compounds of this type have a lipid-reducing action.

ML-3000 ([2,2-dimethyl-6-(4-chlorophenyl)-7-phenyl-2,3-dihydro-1 H-pyrrolizine-5 yl]-acetic acid) of Formula (la)

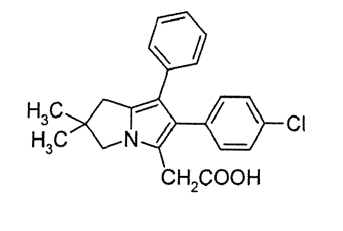

is a non-antioxidant balanced dual inhibitor of COX and 5-LOX (Laufer S, Tries S, Augustin J, Dannhardt G. Pharmacological profile of a new pyrrolizine derivative inhibiting the enzymes cyclo-oxygenase and 5-lipoxygenase. Arzneimittelforschung 1994; 44:629-36). The drug, also known as licofelone, is a non-selective inhibitor of COX, inhibiting both COX-1 and COX-2. This drug has analgesic, antipyretic and anti-inflammatory activity, and has been demonstrated to have potent anti-inflammatory action in a number of animal models including carrageenan-induced paw oedema in the rat, and rat adjuvant arthritis (Laufer S, Tries S, Augustin J, Elsasser R, Albrecht W, Guserle R, et al. Acute and chronic anti-inflammatory properties of [2,2-dimethyl-6-(4-chlorophenyl)-7-phenyl-2,3-dihydro-1 H-pyrrolizine-5-yl]-acetic acid. Arzneimittelforschung 1995; 45:27-32). WO-A 03/020267 describes that ML3000 has chondroprotective effects. Further, it has been reported that ML3000 shows excellent gastrointestinal tolerability (Laufer S, Tries S, Augustin I, Elsäßer R, Algate DR, Atterson PR, Munt PL (1994) Gastrointestinal Tolerance of [2,2-Dimethyl-6-(4-chlorophenyl)-7-phenyl-2,3-dihydro-1 H-pyrrolizine-5-yl]-acetic Acid in the Rat. Arzneim.-Forsch./Drug Res. 44 (II): 1329-1333; Tries S and Laufer S (2001), The pharmacological profile of ML3000: A new pyrrolizine derivative inhibiting the enzymes cyclo-oxygenase and 5-lipoxygenase. Inflammopharmacology 9: 113-124) and has gastroprotective properties (WO-A 03/097041).

One object of the present invention was to provide new medical uses for pyrrolizines.

Surprisingly, it has now been found, in fact, that certain annellated pyrrolizines, such as ML-3000 (licofelone), are also directly effective in the prevention and treatment of neoplasia, as well as in the general induction of apoptosis in cells, in particular mammalian cells. This effect does not depend on the suppression of the synthesis of inflammatory mediators, as it was shown to be not reversed by addition of exogenous COX and 5-LOX products, but rather seems to be caused by a direct stimulation of the treated cells' readiness to undergo apoptosis. Furthermore, licofelone was shown to be effective also in neoplastic cells negative for COX-2 and in other cell types resistant to treatment with conventional chemotherapeutics (multi-drug resistance).

### SUMMARY OF THE INVENTION

Thus, the present invention relates to the use of annellated pyrrole compounds represented by the general formula (I): 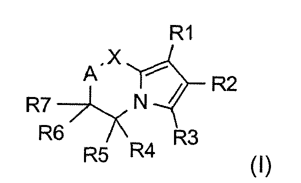 wherein
X represents CR8R9, S, O, NR12 or C(O);
A represents CR10R11 or a bond between X and the atom carrying radicals R6 and R7;
the first of radicals R1, R2, R3 represents
   aryl, optionally substituted with one or more than one substituents independently selected among the group consisting of halogen, alkyl, halogenoalkyl, alkoxy, aryloxy, halogenoalkoxy, alkylthio, hydroxy, nitro, alkylsulfinyl, alkylsulfonyl, sulfamoyl, N-alkylsulfamoyl, N,N-dialkylsulfamoyl, alkylsulfonamido and alkylsulfon-N-alkylamido; or
   an aromatic or non-aromatic, mono- or bicyclic, optionally benzoannellated, heterocyclic group having 1, 2 or 3 heteroatoms independently selected from N, O and S and optionally being substituted with one or more than one substituents independently selected among the group consisting of halogen, alkyl, halogenoalkyl, alkoxy, aryloxy, halogenoalkoxy, alkylthio, hydroxy, nitro, alkylsulfinyl, alkylsulfonyl, sulfamoyl, N-alkylsulfamoyl, N,N-di-alkylsulfamoyl, alkylsulfonamido and alkylsulfon-N-alkylamido;
the second of radicals R1, R2, R3 represents
   alkyl, optionally substituted with one or more than one substituents independently selected among the group consisting of halogen, cycloalkyl, alkoxy, trifluoromethoxy, hydroxy and trifluoromethyl; cycloalkyl, optionally substituted with one or more than one substituents independently selected among the group consisting of halogen, alkyl, halogenoalkyl, cycloalkyl, alkoxy, halogenoalkoxy and hydroxy; aryl, optionally substituted with one or more than one substituents independently selected among the group consisting of halogen, alkyl, halogenoalkyl, alkoxy, aryloxy, halogenoalkoxy, alkylthio, hydroxy, nitro, alkylsulfinyl, alkylsulfonyl, sulfamoyl, N-alkylsulfamoyl, N,N-dialkylsulfamoyl, alkylsulfonamido and alkylsulfon-N-alkylamido; or an aromatic or non-aromatic, mono- or bicyclic, optionally benzoannellated, heterocyclic group having 1, 2 or 3, heteroatoms independently selected from N, O and S and optionally being substituted with one or more than one substituents independently selected among the group consisting of halogen, alkyl, halogenoalkyl, alkoxy, aryloxy, halogenoalkoxy, alkylthio, hydroxy, nitro, alkylsulfinyl, alkylsulfonyl, sulfamoyl, N-alkylsulfamoyl, N,N-di-alkylsulfamoyl, alkylsulfonamido and alkylsulfon-N-alkylamido;
the third of radicals R1, R2, R3 represents
   H, alkyl, halogenoalkyl, hydroxyalkyl, -CHO, -COOH, halogen, cyano, alkylsulfonyl, sulfamoyl or A'-Y;
   wherein
   A' represents alkylene or alkenylene, optionally substituted with hydroxy or alkoxy;
   Y represents -COOH, SO₃H, OPO(OH)₂, OP(OH)₂, -CHO or tetrazolyl; or
the second and the third of radicals R1, R2, R3 represent,
   together with the atom they are attached to, saturated or unsaturated cycloalkyl;
R4-R11, which may be the same or different, represent
   hydrogen, alkyl, hydroxyalkyl, alkoxyalkyl, hydroxy, COOH or acyloxy, where vicinal radicals may also represent bonds or geminal radicals, together with the C atom they are attached to, may also represent carbonyl or cycloalkyl;
R12 represents hydrogen, alkyl or phenyl,
and physiologically acceptable salts and derivatives thereof,
for the prevention and/or treatment of neoplasia.

It is to be understood here that in any embodiment of the invention where the compound of formula (I) comprises at least one site of asymmetry, e. g. an asymmetric carbon atom, the invention extends to all the optical isomers thereof, as well as to racemates and to isomer mixtures wherein any isomer or any number of isomers may be present in any amount.

The term "neoplasia" refers to any non-physiological tissue growth and comprises in particular both benign and malign growths including polyps, tumours and their established metastases, as well as the precursors of such growths, including dysplastic cells and disseminating tumour cells; or, in other words, to any condition characterized by the occurrence of neoplastic cells. Thus, neoplasia comprises in particular dysplasia and neoplastic disease.

The terms "dysplastic cell" and "dysplasia" (a condition characterized by the occurrence of dysplastic cells) are herein used to denote an abnormality in the appearance of a cell, in particular of an epithelial cell, indicative of an early, but genetically rooted and thus generally irreversible step in neoplastic transformation; it is therefore a pre-tumourous change which is restricted to the original tissue of the dysplastic cell, particularly the epithelial layer when the dysplastic cell is an epithelial cell, not (yet) invading into the deeper or further tissue.

A "neoplastic cell" is herein said to be so as soon as it enters a genetic and/or physiological state potentially enabling its unlimited growth, or otherwise typical for neoplastic disease, independent of the stage of clinically evident manifestation of the disease; e. g. a pre-malignant or malignant cell, a dysplastic cell or a tumour cell, e. g. a primary tumour cell, a disseminating tumour cell or a tumour cell forming an established metastasis.

"Neoplastic disease" is herein used to denote any clinically or preclinically manifest form of tissue disease characterized by neoplasia, e. g. a benign tumour or a malign tumour.

The term "treatment of neoplasia" includes partial or total inhibition of neoplasia in a subject, as well as partial or total destruction of the neoplastic cells.

The term "prevention of neoplasia" includes preventing the onset of clinically evident neoplasia, e. g. neoplastic disease, altogether as well as preventing the onset of a preclinically evident stage of neoplasia, e. g. neoplastic disease, in subjects at risk. Also intended to be encompassed by this definition is the prevention of initiation for malignant cells or to arrest or reverse the progression of pre-malignant cells to malignant cells. This includes prophylactic treatment of those at risk of developing neoplastic disease.

As used herein, the term "subject" for purposes of treatment includes mammalian, preferably human, subject who has or may have any form of neoplasia, e. g. neoplastic disease. For methods of prevention, the subject is any animal, preferably mammal and more preferably human, subject at risk for developing neoplasia, in particular neoplastic disease, preferably neoplastic disease derived from an epithelial cell.

As used herein, the term "being at risk" refers to having a risk that is higher, preferably significantly higher, of developing neoplasia than the majority of its peerage group defined by basic medical factors such as age, gender, weight, etc., well-known to the skilled person. The subject may be at risk due to exposure to carcinogenic agents, e. g. ionizing radiation or chemical mutagens, genetic predisposition to develop neoplastic disease, and the like, in particular due to smoking or predisposition for FAP.

The term "alkyl, alkoxy etc." includes linear or branched alkyl groups, such as CH₃, C₂H₅, n-propyl, CH(CH₃)₂, n-butyl, CH(CH₃)-C₂H₅, isobutyl, C(CH₃)₃, n-pentyl or n-hexyl, in particular CH₃, C₂H₅ or CH(CH₃)₂, preferably having - unless otherwise stated - 1 to 8, in particular 1 to 6 and more preferably 1 to 4 carbon atoms; as a substituent of a radical R1 to R12 "alkyl, alkoxy etc." preferably comprises 1 to 4 carbon atoms.

Substituted "alkyl, alkoxy etc." includes in particular:
halogenoalkyl, i.e., alkyl, which is partially or completely substituted with fluoro, chloro, bromo and/or iodo, e.g. CH₂F, CHF₂, CF₃, CH₂Cl, 2-fluoroethyl, 2-chloroethyl or 2,2,2-trifluoroethyl; as a substituent of a radical R1 to R12 halogenoalkyl preferably means CHF₂ and especially CF₃;
halogenoalkoxy, i.e., alkoxy, which is partially or completely substituted with fluoro, chloro, bromo and/or iodo, e.g. halogenoalkoxy residues corresponding to the afore-mentioned halogenoalkyl residues; as a substituent of a radical R1 to R12 halogenoalkoxy preferably means OCHF₂ and especially OCF₃;
alkoxyalkyl, i.e., alkyl substituted by alkoxy, e.g. -CH₂-OCH₃ or 2-methoxyethyl;
hydroxyalkyl, i.e., alkyl which is - preferably mono - substituted by hydroxy, e.g., hydroxymethyl or 2-hydroxyethyl;
trifluoromethylalkyl, i.e. alkyl, which is - preferably mono - substituted by trifluoromethyl, e.g., the residues as described in respect of hydroxyalkyl which are substituted with trifluoromethyl instead of hydroxy;
trifluoromethoxyalkyl, i.e. alkyl, which is - preferably mono - substituted by trifluoromethoxy, e.g., the residues as described in respect of hydroxyalkyl which are substituted with trifluoromethoxy instead of hydroxy;
cycloalkylalkyl, i.e., alkyl, which is - preferably mono - substituted by cycloalkyl, e.g. the residues as described in respect of hydroxyalkyl which are substituted with cyclopropyl, cyclobutyl, cyclopentyl or cyclohexyl instead of hydroxy.

The term "cycloalkyl" includes mono- or bicyclic alkyl groups, such as cyclopropyl, cyclobutyl, cyclopentyl, cyclohexyl, etc., preferably having - unless otherwise stated - 3 to 9, in particular 3 to 7 and more preferably 5 or 6 carbon atoms.

The term "alkylene" includes linear or branched alkylene groups, such as methylene and ethylene, preferably having - unless otherwise stated - 1 to 8, in particular 1 to 6 and more preferably 1 to 4 carbon atoms. If alkylene is substituted with hydroxyl or alkoxy, monosubstitution is preferred.

The term "alkenylene" includes linear or branched, mono- or polyunsaturated alkylene groups, such as ethenylene, preferably having - unless otherwise stated - 2 to 8, in particular 2 to 6 and more preferably 2 to 4 carbon atoms. If alkenylene is substituted with hydroxyl or alkoxy, monosubstitution is preferred.

Acyloxy means -OCOR, wherein R represents alkyl or aryl. Preferred examples are acetyloxy and benzoyloxy.

-COOAlkyl means alkoxycarbonyl, such as CO-OCH₃, CO-OC₂H₅, CO-OCH₂-C₂H₅, CO-OCH(CH₃)₂, n-butoxycarbonyl, CO-OCH(CH₃)-C₂H₅, CO-OCH₂-CH(CH₃)₂, CO-OC(CH₃)₃, in particular CO-OCH₃, CO-OC₂H₅, CO-OCH(CH₃)₂ or CO-OCH₂-CH(CH₃)₂.

-COOAlkPhenyl means an alkoxycarbonyl group which is substituted on the alkyl moiety with phenyl, such as benzyloxycarbonyl.

Alkylthio means -S-Alkyl and is also referred to as alkylsulfanyl or alkylmercapto, such as SCH₃, SC₂H₅, SCH₂-C₂H₅, SCH(CH₃)₂, n-butylthio, 1-methylpropylthio, 2-methylpropylthio, SC(CH₃)₃. Methylthio is preferred.

Alkylsulfinyl means -S(O)-Alkyl and is also referred to as alkylsulfoxo, such as SO-CH₃, SO-C₂H₅, n-propylsulfinyl, 1-methylethylsulfinyl, n-butylsulfinyl, 1-methylpropylsulfinyl, 2-methylpropylsulfinyl, 1,1-dimethylethylsulfinyl. Methylsulfinyl is preferred.

Alkylsulfonyl means -S(O)₂-Alkyl and is also referred to as alkylsulfone, such as SO₂-CH₃, SO₂-C₂H₅, n-propylsulfonyl, SO₂-CH(CH₃)₂, n-butylsulfonyl, 1-methylpropylsulfonyl, 2-methylpropylsulfonyl, SO₂-C(CH₃)₃. Methylsulfonyl is preferred.

Sulfamoyl means -S(O)₂NH₂ and is also referred to as amidosulfonyl or sulfonic acid amid.

N-Alkylsulfamoyl means mono-substituted sulfamoyl -S(O)₂NH-Alkyl, e.g.-S(O)₂NH-CH₃.

N,N-Dialkylsulfamoyl means di-substituted sulfamoyl -S(O)₂N-(Alkyl)₂, wherein the N-bounded alkyl residues may be the same or different, e.g. -S(O)₂N(CH₃)₂.

Alkylsulfonamido means -NHS(O)₂-Alkyl, such as NHSO₂-CH₃, NHSO₂-C₂H₅, n-propylsulfonamido, NHSO₂-CH(CH₃)₂, n-butylsulfonamido, 1-methylpropylsulfonamido, 2-methylpropylsulfonamido, NHSO₂-C(CH₃)₃. Methylsulfonamido is preferred.

Alkylsulfon-N-alkylamido means -N(Alkyl)S(O)₂-Alkyl, wherein the N- and the S-bounded alkyl residues may be the same or different, e.g. N(CH₃)SO₂-CH₃.

Carbonyl, CHO, -COOH, -SO₃H means >C=O, formyl, carboxy, carboxycarbonyl and sulfo, respectively.

"Aryl" preferably means naphthyl and in particular phenyl.

The term "halogen" includes a fluoro, chloro, bromo or iodo atom. Usually fluoro and chloro, and in some cases also bromo are preferred.

"Heterocyclic residues" include in particular 5- or 6-membered heterocyclic residues which may be aromatic or non-aromatic, mono- or bicyclic, and/or benzoannellated. Examples are nitrogen-containing heterocyclic residues, such as pyrrolyl, imidazolyl, pyrazolyl, pyridazinyl, pyrazinyl, indolyl, chinolinyl, especially pyridyl, pyrimidyl and isochinolinyl. The aromatic residues also include heterocyclic residues which contain an oxygen atom or a sulfur atom, such as thienyl, benzothienyl, furanyl and especially benzofuranyl. Also included are heterocyclic residues which contain 2 or more than 2 different heteroatoms, such as thiazolyl, isothiazolyl, thiadiazolyl, isoxazolyl and oxazolyl. Thienyl, pyridyl and thiazolyl are preferred aromatic heterocyclic residues. Non-aromatic residues include nitrogen-containing heterocyclic residues, such as pyrrolidinyl, piperidinyl and piperazinyl. This also includes heterocyclic residues which contain 2 or more than 2 different heteroatoms, such as morpholinyl.

Substituted residues, in particular alkyl, cycloalkyl, aryl and heteroaryl, are preferably mono-, di- or tri-substituted.

The [α]-annelland may be 6- or especially 5-membered, heterocyclic or especially alicyclic, if alicyclic, then unsaturated or especially saturated, and/or substituted or unsubstituted.

The [α]-annellated pyrrole compounds of Formula (I) include in particular those wherein X represents CR8R9 and A represents a bond between X and the atom carrying radicals R6 and R7 (pyrrolizines); X represents CR8R9 and A represents CR10R11 (indolizines); X represents NR12 and A represents a bond between X and the atom carrying radicals R6 und R7 (pyrrolo[1,2-a]imidazoles); X represents S and A represents a bond between X and the atom carrying radicals R6 and R7 (pyrrolo[2,1-b]thiazoles); X represents S and A represents CR10R11 (pyrrolo[2,1-b]1,3-thiazines); X represents O and A represents CR10R11 (pyrrolo[2,1-b]1,3-oxazines); X represents O and A represents a bond between X and the atom carrying radicals R6 and R7 (pyrrolo[2,1-b]oxazoles), residues not mentioned having the meanings given above.

If the [α]-annelland is a 5-membered unsaturated residue, especially R4 and R6 represent a bond, such as, e.g., in pyrrolizine, pyrrolo[2,1-b]imidazole and pyrrolo[2,1-b]thiazole. If the [α]-annelland is a 6-membered unsaturated residue, especially R4 and R6, such as, e.g., in pyrrolo[2,1-b]1,3-thiazine, pyrrolo[2,1-b]1,3-oxazine or 5,6-dihydroindolizine, and optionally also R8 and R10, such as, e.g., in indolizine, represent a bond.

Without being bound to a specific [α]-annelland, according to a particular embodiment of the invention, R4-R11 which may be the same or different represent hydrogen or alkyl. According to a further particular embodiment of the invention, at least one of radicals R4, R5, R6 and R7 represents hydroxyalkyl, in particular hydroxymethyl, and the remaining radicals R4, R5, R6 and R7 independently represent H or alkyl. According to this embodiment it is preferred that R4 is hydroxyalkyl, in particular hydroxymethyl, and R5 is H or alkyl, and R6, R7 independently are H or alkyl. According to a further particular embodiment of the invention, one of radicals R8 and R9 represents H, alkyl, hydroxyalkyl or alkoxyalkyl and the other represents hydroxyl, alkoxy, carboxyl or acyloxy, or R8 and R9 together with the C atom they are attached to, represent a carbonyl group.

6,7-Dihydro-5H-pyrrolizines are especially useful, i.e. compounds of Formula (I), wherein X represents CR8R9, A represents a bond between X and the atom carrying radicals R6 und R7, and R4, R5, R6, R7, R8, R9 which may be the same or different, have the meaning as given above and preferably represent hydrogen or alkyl. 6,7-Dihydro-5H-pyrrolizine wherein R4 to R9 are hydrogen or at least one or two of radicals R4 to R9, for instance R6 and/or R7, represent alkyl, in particular methyl, are especially preferred.

According to an important aspect of the present invention, compounds of Formula (I), wherein the first and the second of radicals R1, R2, R3, preferably R1 and R2, independently represent an π-electron-rich system selected from aryl and aromatic heterocyclic residues, in particular phenyl, optionally substituted with one or more than one substituents that in particular are independently selected among the group consisting of halogen, alkyl and halogenoalkyl, in particular CF₃, R1 being preferably unsubstituted phenyl and R2 being preferably 4-substituted phenyl, are especially useful.

According to a further important aspect of the invention, compounds of Formula (I), wherein the third of radicals R1, R2, R3, preferably R3, represents an acidic residue such as COOH or A'-Y, wherein Y is COOH and A' preferably represents alkylene, or represents a precursor of an acidic residue such as A'-Y, wherein Y is tetrazolyl, are especially useful.

The use of [6-(4-chlorophenyl)-2,2-dimethyl-7-phenyl-2,3-dihydro-1 H-pyrrolizine-5-yl]-acetic acid (ML3000) represented by formula (Ia): 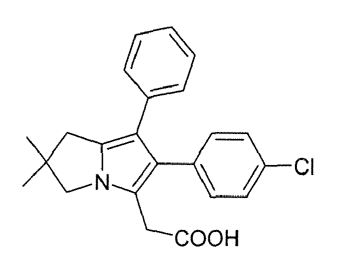 or its physiologically acceptable salts and derivatives, e.g., physiologically hydrolysable esters, is especially preferred.

Physiologically acceptable salts include acid or base addition salts.

Acid addition salts are, for instance, salts of compounds of Formula (I) with inorganic acids, such as hydrochloric acid, sulfuric acid, nitric acid or phosphoric acid, or with organic acids, in particular carboxylic acids, e.g. acetic acid, tartaric acid, lactic acid, citric acid, maleic acid, amygdalic acid, ascorbic acid, maleic acid, fumaric acid, gluconic acid or sulfonic acid, e. g. methanosulfonic acid, phenylsulfonic acid and toluenesulfonic acid, and the like.

Base addition salts are, for instance, salts of compounds of Formula (I) with inorganic bases, such as sodium or potassium hydroxide or with organic bases, such as mono-, di- or triethanolamine, and the like.

Physiologically acceptable derivatives include in particular prodrugs of the compounds of formula (I) which are reconverted in vivo to the compounds of formula (I) or an active form thereof (metabolite). Examples are hydrolysable esters of the compounds of formula (I) wherein the third of radicals R1, R2, R3 represents an acidic residue, e.g. alkyl (the third of radicals R1, R2, R3 comprising the functionality COOAlkyl), aralkyl (the third of radicals R1, R2, R3 comprising the functionality COOAlkaryl, e.g., COOAlkPhenyl), pivaloyloxymethyl, acetoxymethyl, phthalidyl, indanyl and methoxymethyl esters thereof.

In a preferred embodiment of the invention, the neoplasia to be prevented and/or treated is a tumour selected from the group consisting of papilloma, carcinoma, adenoma, adenocarcinoma, soft tissue sarcoma, melanoma, fibroma, fibrosarcoma, lipoma, liposarcoma, leiomyoma, leiomyosarcoma, rhabdomyoma, rhabdomyosarcoma, mesothelioma, angioma, angiosarcoma, osteoma, osteosarcoma, chondroma, chondrosarcoma, glioma, lymphoma and leukaemia, their precursors and metastases, preferably from the group consisting of papilloma, carcinoma, adenoma and adenocarcinoma, their precursors, e.g. disseminating cells, and established metastases, more preferably from the group consisting of papilloma, carcinoma, adenoma and adenocarcinoma, their precursors, e.g. disseminating cells, and established metastases.

In a preferred embodiment of the invention, the neoplasia to be prevented and/or treated is a fast-growing malignancy, in particular a malignancy whose cells display an inclination to necrotic cell death, e. g. an inclination to necrotic cell death in reaction to immunological, physiological, chemical, physical or radiation-induced stress or damage.

It is particularly preferred if the tumour to be prevented and/or treated is a human tumour of epithelial origin (carcinoma), more preferred if it is a human airway tumour of epithelial origin, e. g. a trachea, bronchus or lung tumour of epithelial origin, e.g. a lung adenocarcinoma, squamous cell carcinoma or small cell carcinoma, or if it is a human colorectal tumour of epithelial origin, e. g. a colon carcinoma, and most preferred if the tumour to be treated is a tumour, e. g. an adenoma or carcinoma, of adenomatous polyposis, in particular of familial adenomatous polyposis (FAP), the term "FAP" here referring to the classical form as well as to Gardner's syndrome and to Turcot's syndrome. In another particularly preferred embodiment, the tumour is a tumour of a cell or tissue, preferably an endothelial tissue, e. g. a skin or mucosa tissue, which expresses the epithelial growth factor receptor (EGFR).

The term "tumour of" is herein used to refer to a tumour causally connected to the condition named, preferably a tumour that arises from tissue directly affected by the condition, more preferably a tumour derived from tumourigenic progression of cells affected by the condition.

In a further preferred embodiment of the invention, the neoplastic cell exhibits resistance to conventional chemotherapeutics, preferably the MDR (multidrug resistance) phenotype. The term "resistance to" is herein used to refer to any significant increase in the required dosage of a chemotherapeutic agent, compared to the parental tissue which the neoplastic growth is derived from. The term "multidrug resistance" is used herein to refer to resistance caused by overexpression of the MDR protein (gp170), which is capable of clearing the cell of various typed of chemotherapeutic agents, such as, e. g., doxorubicin.

In a further preferred embodiment of the invention, the neoplastic cell is negative for expression of COX-2 as defined above. The term "negative for expression" is herein used to denote absence of the COX-2 protein, e. g. absence of the COX-2 protein in a Western blot, of the mRNA transcript encoding the same, e. g. absence of the COX-2 mRNA transcript in a Northern blot or RT-PCR assay, or absence of the biochemical activity of COX-2, preferably absence of the COX-2 protein.

According to a particular embodiment of the present invention the use of a compound of formula (I), a physiologically acceptable salt or derivative thereof, for treating or preventing neoplasia does not involve the use of a compound selected from Taisho NS-398, meloxicam, floculide, Merck MK-966, Merck L-752,860 or a compound of Formula (II) 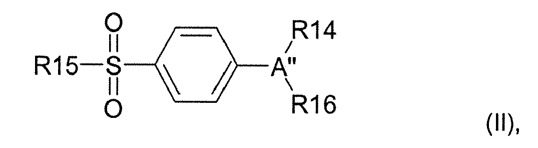 wherein A" is a substituent selected from partially unsaturated or unsaturated heterocyclyl and partially unsaturated or unsaturated carbocyclic rings;
wherein R14 is at least one substituent selected from heterocyclyl, cycloalkyl, cycloalkenyl and aryl, wherein R14 is optionally substituted at a substitutable position with one or more radicals selected from alkyl, haloalkyl, cyano, carboxyl, alkoxycarbonyl, hydroxyl, hydroxyalkyl, haloalkoxy, amino, alkylamino, arylamino, nitro, alkoxyalkyl, alkylsulfinyl, halo, alkoxy and alkylthio;
wherein R15 is methyl or amino; and
wherein R16 is a radical selected from hydrido, halo, alkyl, alkenyl, alkynyl, oxo, cyano, carboxyl, cyanoalkyl, heterocyclyloxy, alkyloxy, alkylthio, alkylcarbonyl, cycloalkyl, aryl, haloalkyl, heterocyclyl, cycloalkenyl, aralkyl, heterocyclylalkyl, acyl, alkylthioalkyl, hydroxyalkyl, alkoxycarbonyl, arylcarbonyl, aralkylcarbonyl, aralkenyl, alkoxyalkyl, arylthioalkyl, aryloxyalkyl, aralkylthioalkyl, aralkoxyalkyl, alkoxyaralkoxyalkyl, alkoxycarbonylalkyl, aminocarbonyl, aminocarbonylalkyl, alkylaminocarbonyl, N-arylaminocarbonyl, N-alkyl-N-arylaminocarbonyl, alkylaminocarbonylalkyl, carboxyalkyl, alkylamino, N-arylamino, N-aralkylamino, N-alkyl-N-aralkylamino, N-alkyl-N-arylamino, aminoalkyl, alkylaminoalkyl, Narylaminoalkyl, N-aralkylaminoalkyl, N-alkyl-Naralkylaminoalkyl, N-alkyl-N-arylaminoalkyl, aryloxy, aralkoxy, arylthio, aralkylthio, alkylsulfinyl, alkylsulfonyl, aminosulfonyl, alkylaminosulfonyl, N-arylaminosulfonyl, arylsulfonyl, N-alkyl-N-arylaminosulfonyl; or a pharmaceutically-acceptable salt thereof.

According to WO-A 96/41626 said compounds are said to be selective cyclooxygenase-2 inhibitors and useful in combination with 5-lipoxygenase inhibitors for treating a variety of disorders, the term "COX-2 selective" being used to refer to compounds which selectively inhibit COX-2 over COX-1, preferably with an IC₅₀ for COX-2 of less than about 0.5 µM and also with a selectivity ratio of COX-2 inhibition over COX-1 inhibition of at least 50, and more preferably of at least 100; it is particularly preferred that they have an IC₅₀ for COX-1 of greater than about 1 µM, in particular of greater than 20 µM.

Particular examples of said selective COX-2 inhibitors of formula (II) include those wherein A" is selected from oxazolyl, isoxazolyl, thienyl, furyl, dihydrofuryl, pyrrolyl, pyrazolyl, thiazolyl, phenyl, imidazolyl, isothiazolyl, benzofuryl, cyclopentenyl, cyclopentadienyl, and pyridyl;
wherein R14 is selected from pyridyl optionally substituted at a substitutable position with one or more methyl radicals, and phenyl optionally substituted at a substitutable position with one or more radicals selected from methyl, ethyl, isopropyl, butyl, tert-butyl, isobutyl, pentyl, hexyl, fluoromethyl, difluoromethyl, trifluoromethyl, cyano, carboxyl, methoxycarbonyl, ethoxycarbonyl, hydroxyl, hydroxymethyl, trifluoromethoxy, amino, N-methylamino, N,N-dimethylamino, N-ethylamino, N,N-dipropylamino, Nbutylamino, N-methyl-N-ethylamino, phenylamino, methoxymethyl, methylsulfinyl, fluoro, chloro, bromo, methoxy, ethoxy, propoxy, n-butoxy, pentoxy, and methylthio; wherein R15 is methyl or amino; and wherein R16 is a radical selected from hydrido, oxo, cyano, carboxyl, methoxycarbonyl, ethoxycarbonyl, carboxypropyl, carboxymethyl, carboxyethyl, cyanomethyl, fluoro, chloro, bromo, methyl, ethyl, isopropyl, butyl, tert-butyl, isobutyl, pentyl, hexyl, difluoromethyl, trifluoromethyl, pentafluoroethyl, heptafluoropropyl, difluoroethyl, difluoropropyl, methoxy, ethoxy, propoxy, n-butoxy, pentoxy, cyclohexyl, phenyl, pyridyl, thienyl, thiazolyl, oxazolyl, furyl, pyrazinyl, hydroxylmethyl, hydroxylpropyl, benzyl, formyl, phenylcarbonyl, methoxymethyl, furylmethyloxy, aminocarbonyl, N-methylaminocarbonyl, N,N-dimethylaminocarbonyl, N,N-dimethylamino, N-ethylamino, N,N-dipropylamino, N-butylamino, N-methyl-N-ethylamino, aminomethyl, N,Ndimethylaminomethyl, N-methyl-N-ethylaminomethyl, benzyloxy, and phenyloxy; or a pharmaceuticallyacceptable salt thereof. Specific examples of said selective COX-2 inhibitors of formula (II) include
5-(4-fluorophenyl)-1-[4-(methylsulfonyl)phenyl]-3-(trifluoromethyl)pyrazole;
4-(4-fluorophenyl)-5-[4-(methylsulfonyl)phenyl]-1-phenyl-3-(trifluoromethyl)pyrazole;
4-(5-(4-chlorophenyl)-3-(4-methoxyphenyl)-1H-pyrazol-1-yl)benzenesulfonamide;
4-(3,5-bis(4-methylphenyl)-1H-pyrazol-1-yl)benzenesulfonamide;
4-(5-(4-chlorophenyl)-3-phenyl-1H-pyrazol-1-yl)benzenesulfonamide;
4-(3,5-bis(4-methoxyphenyl)-1H-pyrazol-1-yl)benzenesulfonamide;
4-(5-(4-chlorophenyl)-3-(4-methylphenyl)-1H-pyrazol-1-yl)benzenesulfonamide;
4-(5-(4-chlorophenyl)-3-(4-nitrophenyl)-1H-pyrazo)-1-yl)benzenesulfonamide;
4-(5-(4-chlorophenyl)-3-(5-chloro-2-thienyl)-1H-pyrazol-1-yl)benzenesulfonamide;
4-(4-chloro-3,5-diphenyl-1H-pyrazol-1-yl)benzenesulfonamide;
4-[5-(4-chlorophenyl)-3-(trifluoromethyl)-1H-pyrazol-1-yl]benzenesulfonamide;
4-[5-phenyl-3-(trifluoromethyl)-H-pyrazol-1-yl]benzenesulfonamide;
4-[5-(4-fluorophenyl)-3-(trifluoromethyl)-1H-pyrazol-1-yl]benzenesulfonamide;
4-[5-(4-methoxyphenyl)-3-(trifluoromethyl)-1H-pyrazol-1-yl]benzenesulfonamide;
4-[5-(4-chlorophenyl)-3-(difluoromethyl)-1H-pyrazol-1-yl]benzenesulfonamide;
4-[5-(4-methylphenyl)-3-(trifluoromethyl)-1H-pyrazol-1yl]benzenesulfonamide;
4-[4-chloro-5-(4-chlorophenyl)-3-(trifluoromethyl)-1Hpyrazol-1-yl]benzenesulfonamide;
4-[3-(difluoromethyl)-5-(4-methylphenyl)-1H-pyrazol-1-yl]benzenesulfonamide;
4-[3-(difluoromethyl)-S-phenyl-1H-pyrazol-1-yl]benzenesulfonamide;
4-[3-(difluoromethyl)-5-(4-methoxyphenyl)-1H-pyrazol-1-yl]benzenesulfonamide;
4-[3-cyano-5-(4-fluorophenyl)-1H-pyrazol-1-yl]benzenesulfonamide;
4-[3-(difluoromethyl)-5-(3-fluoro-4-methoxyphenyl)-1H-pyrazol-1-yl]benzenesulfonamide;
4-[5-(3-fluoro-4-methoxyphenyl)-3-(trifluoromethyl)-1H-pyrazol-1-yl]benzenesulfonamide;
4-[4-chloro-5-phenyl-1H-pyrazol-1-yl]benzenesulfonamide;
4-[5-(4-chlorophenyl)-3-(hydroxymethyl)-1H-pyrazol-1-yl]benzenesulfonamide;
4-[5-(4-(N,N-dimethylamino)phenyl)-3-(trifluoromethyl)-IHpyrazol-I-yl]benzenesulfonamide;
5-(4-fluorophenyl)-6-[4-(methylsulfonyl)phenyl]spiro[2.4]hept-5-ene;
4-[6-(4-fluorophenyl)spiro[2.4]hept-5-en-5-yl]benzenesulfonamide;
6-(4-fluorophenyl)-7-[4(methylsulfonyl)phenyl]spiro[3.4]oct-6-ene;
5-(3-chloro-4-methoxyphenyl)-6-[4(methylsulfonyl)phenyl]spiro[2.4]hept-5-ene;
4-[6-(3-chloro-4-methoxyphenyl)spiro[2.4]hept-5-en-5yl]benzenesulfonamide;
5-(3,5-dichloro-4-methoxyphenyl)-6-[4-(methylsulfonyl)phenyl]spiro[2.4]hept-5-ene;
5-(3-chloro-4-fluorophenyl)-6-[4-(methylsulfonyl)phenyl]spiro[2.4]hept-5-ene;
4-[6-(3,4-dichlorophenyl)spiro[2.4]hept-5-en-5yl]benzenesulfonamide;
2-(3-chloro-4-fluorophenyl)-4-(4-fluorophenyl)-5-(4-methylsulfonylphenyl)thiazole;
2-(2-chlorophenyl)-4-(4-fluorophenyl)-5-(4-methylsulfonylphenyl)thiazole;
5-(4-fluorophenyl)-4-(4-methylsulfonylphenyl)-2-methylthiazole;
4-(4-fluorophenyl)-5-(4-methylsulfonylphenyl)-2-trifluoromethylthiazole;
4-(4-fluorophenyl)-5-(4-methylsulfonylphenyl)-2-(2-thienyl)thiazole;
4-(4-fluorophenyl)-5-(4-methylsulfonylphenyl)-2-benzylaminothiazole;
4-(4-fluorophenyl)-5-(4-methylsulfonylphenyl)-2-(1-propylamino)thiazole;
2-[(3,5-dichlorophenoxy)methyl)-4-(4-fluorophenyl)-5-[4-(methylsulfonyl)phenyl]-thiazole;
5-(4-fluorophenyl)-4-(4-methylsulfonylphenyl)-2-trifluoromethylthiazole;
1-methylsulfonyl-4-[1,1-dimethyl-4-(4-fluorophenyl)cyclopenta-2,4-dien-3-yl]benzene;
4-[4-(4-fluorophenyl)-1,1-dimethylcyclopenta-2,4-dien-3-yl]benzenesulfonamide;
5-(4-fluorophenyl)-6-[4-(methylsulfonyl)phenyl]spiro[2.4]hepta-4,6-diene;
4-[6-(4-fluorophenyl)spiro[2.4]hepta-4,6-dien-5-yl]benzenesulfonamide;
6-(4-fluorophenyl)-2-methoxy-5-[4-(methylsulfonyl)phenyl]pyridine-3-carbonitrile;
2-bromo-6-(4-fluorophenyl)-5-[4-(methylsulfonyl)phenyl]pyridine-3-carbonitrile;
6-(4-fluorophenyl)-5-[4-(methylsulfonyl)phenyl]-2-phenylpyridine-3-carbonitrile;
4-[2-(4-methylpyridin-2-yl)-4-(trifluoromethyl)-1H-imidazol-1-yl]benzenesulfonamide;
4-[2-(5-methylpyridin-3-yl)-4-(trifluoromethyl)-1Himidazol-1-y1benzenesulfonamide;
4-[2-(2-methylpyridin-3-yl)-4-(trifluoromethyl)-1H-imidazol-1-yl]benzenesulfonamide;
3-[1-[4-(methylsulfonyl)phenyl]-4-(trifluoromethyl)-1H-imidazol-2-yl]pyridine;
2-[1-[4-(methylsulfonyl)phenyl-4-(trifluoromethyl)-1H-imidazol-2-yl]pyridine;
2-methyl-4-[1-[4-(methylsulfonyl)phenyl-4(trifluoromethyl)-1H-imidazol-2-yl]pyridine;
2-methyl-6-[1-[4-(methylsulfonyl)phenyl-4-(trifluoromethyl)-1H-imidazol-2-yl]pyridine;
4-[2-(6-methylpyridin-3-yl)-4-(trifluoromethyl)-1H-imidazol-1-yl]benzenesulfonamide;
2-(3,4-difluorophenyl)-1-[4-(methylsulfonyl)phenyl]-4(trifluoromethyl)-1H-imidazole;
4-[2-(4-methylphenyl)-4-(trifluoromethyl)-1H-imidazol-1-yl]benzenesulfonamide;
2-(4-chlorophenyl)-1-[4-(methylsulfonyl)phenyl]-4-methyl-1H-imidazole;
2-(4-chlorophenyl)-1-[4-(methylsulfonyl)phenyl]-4-phenyl-1H-imidazole;
2-(4-chlorophenyl)-4-(4-fluorophenyl)-1-[4-(methylsulfonyl)phenyl]-1H-imidazole;
2-(3-fluoro-4-methoxyphenyl)-I-[4-(methylsulfonyl)phenyl-4-(trifluoromethyl)-1H-imidazole;
1-[4-(methylsulfonyl)phenyl]-2-phenyl-4-trifluoromethyl-1H-imidazole;
2-(4-methylphenyl)-1-[4-(methylsulfonyl)phenyl]-4-trifluoromethyl-1H-imidazole;
4-[2-(3-chloro-4-methylphenyl)-4-(trifluoromethyl)-1H-imidazol-I-yl]benzenesulfonamide;
2-(3-fluoro-5-methylphenyl)-1-[4-(methylsulfonyl)phenyl]-4-(trifluoromethyl)-1H-imidazole;
4-[2-(3-fluoro-5-methylphenyl)-4-(trifluoromethyl)-1H-imidazol-1-yl]benzenesulfonamide;
2-(3-methylphenyl)-1-[4-(methylsulfonyl)phenyl]-4-trifluoromethyl-1H-imidazole;
4-[2-(3-methylphenyl)-4-trifluoromethyl-1H-imidazol-1-yl]benzenesulfonamide;
1-[4-(methylsulfonyl)phenyl]-2-(3-chlorophenyl)-4-trifluoromethyl-1H-imidazole;
4-[2-(3-chlorophenyl)-4-trifluoromethyl-H-imidazol-1-yl]benzenesulfonamide;
4-[2-phenyl-4-trifluoromethyl-1H-imidazol-1yl]benzenesulfonamide;
4-[2-(4-methoxy-3-chlorophenyl)-4-trifluoromethyl-1H-imidazol-l-yl]benzenesulfonamide;
1-allyl-4-(4-fluorophenyl)-3-[4-(methylsulfonyl)phenyl]-5-(trifluoromethyl)-1H-pyrazole;
4-[1-ethyl-4-(4-fluorophenyl)-5-(trifluoromethyl)-1H-pyrazol-3-yl]benzenesulfonamide;
N-phenyl-[4-(4-luorophenyl)-3-[4-(methylsulfonyl)phenyl]-5-(trifluoromethyl)-1H-pyrazol-1-yl]acetamide;
ethyl[4-(4-fluorophenyl)-3-[4-(methylsulfonyl)phenyl]-5-(trifluoromethyl)-1H-pyrazol-1-yl]acetate;
4-(4-fluorophenyl)-3-[4-(methylsulfonyl)phenyl]-1-(2-phenylethyl)-1H-pyrazole;
4-(4-fluorophenyl)-3-[4-(methylsulfonyl)phenyl]-1-(2phenylethyl)-5-(trifluoromethyl)-pyrazole;
1-ethyl-4-(4-fluorophenyl)-3-[4-(methylsulfonyl)phenyl]-5-(trifluoromethyl)-1H-pyrazole;
5-(4-fluorophenyl)-4-(4-methylsulfonylphenyl)-2-trifluoromethyl-1H-imidazole;
4-[4-(methylsulfonyl)phenyl]-5-(2-thiophenyl)-2-(trifluoromethyl)-1H-imidazole;
5-(4-fluorophenyl)-2-methoxy-4-[4-(methylsulfonyl)phenyl]-6-(trifluoromethyl)pyridine;
2-ethoxy-5-(4-fluorophenyl)-4-[4-(methylsulfonyl)phenyl]-6-(trifluoromethyl)pyridine;
5-(4-fluorophenyl)-4-[4-(methylsulfonyl)phenyl]-2-(2-propynyloxy)-6-(trifluoromethyl)-pyridine;
2-bromo-5-(4-fluorophenyl)-4-[4-(methylsulfonyl)phenyl]-6-(trifluoromethyl)pyridine;
4-[2-(3-chloro-4-methoxyphenyl)-4,5difluorophenyl]benzenesulfonamide;
1-(4-fluorophenyl)-2-[4-(methylsulfonyl)phenyl]benzene;5-difluoromethyl-4-(4-methylsulfonylphenyl)-3-phenylisoxazole;
4-[3-ethyl-5-phenylisoxazol-4-yl]benzenesulfonamide4-[5-difluoromethyl-3-phenylisoxazol-4-yl]benzenesulfonamide;
4-[5-hydroxymethyl-3-phenylisoxazol-4-yl]benzenesulfonamide;
4-[5-methyl-3-phenyl-isoxazol-4-yl]benzenesulfonamide;
1-[2-(4-fluorophenyl)cyclopenten-1-yl]-4-(methylsulfonyl)benzene;
1-[2-(4-fluoro-2-methylphenyl)cyclopenten-1-yl]-4-(methylsulfonyl)benzene;
1-[2-(4-chlorophenyl)cyclopenten-1-yl]-4-(methylsulfonyl)benzene;
1-[2-(2,4-dichlorophenyl)cyclopenten-1-yl]-4-(methylsulfonyl)benzene;
1-[2-(4-trifluoromethylphenyl)cyclopenten-1-yl]-4-(methylsulfonyl)benzene;
1-[2-(4-methylthiophenyl)cyclopenten-1-yl]-4-(methylsulfonyl)benzene;
1-[2-(4-fluorophenyl)-4,4-dimethylcyclopenten-1-yl]-4-(methylsulfonyl)benzene;
4-[2-(4-fluorophenyl)-4,4-dimethylcyclopenten-1-yl]benzenesulfonamide;
1-[2-(4-chlorophenyl)-4,4-dimethylcyclopenten-1-yl]-4-(methylsulfonyl)benzene;
4-[2-(4-chlorophenyl)-4,4-dimethylcyclopenten-1yl]benzenesulfonamide;
4-[2-(4-fluorophenyl)cyclopenten-1-yl]benzenesulfonamide;
4-[2-(4-chlorophenyl)cyclopenten-1-yl]benzenesulfonamide;
1-[2-(4-methoxyphenyl)cyclopenten-l-yl]-4-(methylsulfonyl)benzene;
1-[2-(2,3-difluorophenyl)cyclopenten-1-yl]-4-(methylsulfonyl)benzene;
4-[2-(3-fluoro-4-methoxyphenyl)cyclopenten-1-yl]benzenesulfonamide;
1-[2-(3-chloro-4-methoxyphenyl)cyclopenten-1-yl]-4-(methylsulfonyl)benzene;
4-[2-(3-chloro-4-fluorophenyl)cyclopenten-1-yl]benzenesulfonamide;
4-[2-(2-methylpyridin-5-yl)cyclopenten-1yl]benzenesulfonamide;
ethyl2-[4-(4-fluorophenyl)-5-[4-(methylsulfonyl)phenyl]oxazol-2-yl]-2-benzylacetate;
2-[4-(4-fluorophenyl)-5-[4-(methylsulfonyl)phenyl]oxazol-2-yl]aceticacid;
2-(tert-butyl)-4-(4-fluorophenyl)-5-[4-(methylsulfonyl)phenyl]oxazole;
4-(4-fluorophenyl)-5-[4-(methylsulfonyl)phenyl]-2-phenyloxazole;
4-(4-fluorophenyl)-2-methyl-5-[4-(methylsulfonyl)phenyl]oxazole;and
4-[5-(3-fluoro-4-methoxyphenyl)-2-trifluoromethyl-4-oxazolyl]benzenesulfonamide.

Preferably, the use of a compound of formula (I), a physiologically acceptable salt or derivative thereof, for treating or preventing neoplasia does not involve the use of a selective COX-2 inhibitor as defined above.

More preferably, the use of a compound of formula (I), a physiologically acceptable salt or derivative thereof, for treating or preventing neoplasia does not involve the use of any pharmacologically active compound other than a compound of formula (I), a physiologically acceptable salt or derivative thereof.

In a further preferred embodiment of the invention, the use is for the prevention of neoplasia as defined above, preferably in a subject at an increased risk of neoplasia as defined above, more preferably
at an increased risk of developing neoplasia of the colon, preferably due to a familial history of adenomatous polyposis or the onset of a pre-malignant stage of adenomatous polyposis, in particular FAP including Gardner's and Turcet's syndromes, in the subject,
or at an increased risk of developing neoplasia of the airways, in particular due to a history of smoking or to airway metaplasia, preferably to metaplasia of the airway epithelium.

"Metaplasia" herein refers to a situation where cells have changed from their original mature differentiated type into another mature differentiated or partly differentiated cell type, e. g. as an adaptive response to exposure to chronic irritation, or to a pathogen or carcinogen, with or without irreversible genetic changes.

In a further preferred embodiment of the invention, the use is for the treatment of neoplasia as defined above.

In a particularly preferred embodiment of the invention, the treatment of the subject comprises further stimulation of cell death by a conventional method or combination of conventional methods, the conventional methods preferably being selected from the group consisting of irradiation, e. g. external irradiation or administration of radioactive compounds, heating, e. g. microwave treatment of neoplastic tissue, and treatment with a cytostatic agent, e. g. a cytostatic agent selected from the group consisting of alkylating agents, antimetabolites, plant alkaloids and other natural products, cytotoxic antibiotics and related substances, platinum compounds, methylhydrazines, monoclonal antibodies, sensitizers used in photodynamic and/or radiation therapy, further antineoplastic agents, receptor inhibitors, and combinations thereof, examples for each of these groups being given below. It is most preferred if the further stimulation of cell death by a conventional method benefits from an increase in the readiness to undergo apoptosis, e. g. if the further stimulation of cell death by a conventional method results in an inclination to necrotic cell death, e. g. an inclination to necrotic cell death in reaction to immunological, physiological, chemical, physical or radiation-induced stress or damage.

It is particularly preferred if the subject to be treated will not benefit from conventional therapy, e.g. as defined above, in the absence of pharmacotherapy comprising the administration of compounds of Formula (I), physiologically acceptable salts or derivatives thereof, the term "benefit" herein being used to denote the attainment of any or all of the objects of treatment as defined above.

In a further preferred embodiment of the invention, the use is for the induction of apoptosis, preferably in a mammalian cell, more preferably under conditions of insufficient apoptosis. A condition of "insufficient apoptosis" is herein understood to refer to any stage characterized by the appearance of a neoplastic cell, particularly neoplastic disease, or at increased risk of the same as defined above.

Thus, very effective compounds of Formula (I) are those which, or whose physiologically acceptable salts or derivatives, stimulate apoptosis in mammalian cells. The term "stimulate" is used herein to denote any inducing or promoting effect. The term "induce" is used herein to denote any significant increase in the rate of apoptosis in cells treated with compounds of Formula (I), physiologically acceptable salts or derivatives thereof, compared to cells kept under otherwise identical conditions but not treated with compounds of Formula (I), physiologically acceptable salts or derivatives thereof. The term "promote" is used herein to denote any significant increase in the rate of apoptosis in cells treated with compounds of Formula (I), physiologically acceptable salts or derivatives thereof and one or more than one further agent or agents compared to cells kept under otherwise identical conditions including the further agent or agents but not the compounds of Formula (I), physiologically acceptable salts or derivatives thereof.

Suitable compounds can be identified among the compounds of Formula (I), physiologically acceptable salts or derivatives thereof using well-known screening procedures such as high-throughput screening (HTS) procedures. A typical procedure comprises testing the cellular readiness for apoptosis by each of a number of candidate compounds of Formula (I), physiologically acceptable salts or derivatives thereof, and identifying those which have the desired activity.

At a higher level of screening, the suitability for prevention and/or treatment of neoplasia in general, and/or of individual forms of neoplastic disease in particular, may be investigated using animals model known to the skilled artisan.

It is particularly preferred if the use is for the restoration of the natural ability to undergo apoptosis in cells which have partly or wholly lost this ability, more preferably for restoration of the natural ability to undergo apoptosis in neoplastic cells which have fully lost this ability, most preferably in neoplastic cells showing a propensity to necrotic cell death, e. g. a propensity to necrotic cell death in reaction to immunological, physiological, chemical, physical or radiation-induced stress or damage.

The present invention further relates to a pharmaceutical composition for the prevention and/or treatment of neoplasia as defined above, comprising one or more than one compound of Formula (I), a physiologically acceptable salt or derivative thereof, as defined above and one or more cytostatic agents selected from the group consisting of alkylating agents, antimetabolites, plant alkaloids and other natural products, cytotoxic antibiotics and related substances, platinum compounds, methylhydrazines, monoclonal antibodies, sensitizers used in photodynamic and/or radiation therapy, further antineoplastic agents, and combinations thereof. Basically any appropriate cytostatic may be used, depending on the type and stage of the disease, to be decided at the treating physician's or veterinary's discretion. Example for suitable cytostatics comprise alkylating agents, e. g. nitrogen mustard analogues such as cyclophosphamide, chlorambucil, melphalan, chlormethine, ifosfamide, trofosfamide and prednimustine, alkyl sulfonates such as busulfan, treosulfan, mannosulfan, ethylene imines such as thiotepa, triaziquone and carboquone, nitrosoureas such as carmustine, lomustine, semustine, streptozocin, fotemustine, nimustine and ranimustine, epoxides such as etoglucid, and other alkylating agents such as mitobronitol, pipobroman, temozolomide and dacarbazine; antimetabolites, e. g. folic acid analogues such as methotrexate, raltitrexed and pemetrexed, purine analogues such as mercaptopurine, tioguanine, cladribine, fludarabine, clofarabine, pyrimidine analogues such as cytarabine, fluorouracil, tegafur, carmofur, gemcitabine and capecitabine; plant alkaloids and other natural products, e. g. vinca alkaloids and analogues, such as vinblastine, vincristine, vindesine and vinorelbine, podophyllotoxin derivatives such as etoposide and teniposide, colchicine derivatives such as demecolcine, taxanes such as paclitaxel and docetaxel; other plant alkaloids and natural products, e. g. trabectedin; cytotoxic antibiotics and related substances, e. g. actinomycines such as dactinomycin, anthracyclines and related substances such as doxorubicin, daunorubicin, epirubicin, aclarubicin, zorubicin, idarubicin, mitoxantrone, pirarubicin and valrubicin; other cytotoxic antibiotics such as bleomycin, plicamycin and mitomycin; other antineoplastic agents, e. g. platinum compounds such as cisplatin, carboplatin and oxaliplatin, methylhydrazines such as procarbazine, monoclonal antibodies such as edrecolomab, rituximab, trastuzumab, alemtuzumab, gemtuzumab, cetuximab, bevacizumab and panitumumab; sensitizers used in photodynamic and/or radiation therapy, e. g. porfimer sodium, verteporfin, methyl aminolevulinate, aminolevulinic acid, temoporfin, efaproxiral; receptor inhibitors, e. g. gefitinib, erlotinib and tamoxifen; further antineoplastic agents, e. g. amsacrine, asparaginase, altretamine, hydroxycarbamide, lonidamine, pentostatin, miltefosine, masoprocol, estramustine, tretinoin, mitoguazone, topotecan, tiazofurine, irinotecan, alitretinoin, mitotane, pegaspargase, bexarotene, arsenic trioxide, imatinib, denileukin, diftitox, bortezomib, celecoxib, and anagrelide; and combinations of these.

Particularly preferred examples for cytostatic agents to be used in the treatment of FAP and tumours of FAP comprise tamoxifen; doxorubicin, dacarbazine and combinations of these; cyclophosphamide, mitomycin, cisplatin, ifosfamide, etoposide and any combinations of these with each other and/or doxorubicin; and irinotecan.

Particularly preferred examples for cytostatic agents to be used in the treatment of tumours of the airways comprise paclitaxel alone or in combination with carboplatin and/or vinorelbin; gefitinib; gemcitabine, etoposide, irinotecan, mitomycin or fluorouracil alone or in combination with cisplatin; temozolomide; and oxaliplatin.

In particular, the present invention relates to the use of one or more than one compound of Formula (I), a physiologically acceptable salt or derivative thereof as defined herein, for preparing a pharmaceutical composition for treating or preventing neoplasia.

The present invention also relates to methods of treatment or prevention of neoplasia, and pharmaceutical compositions useful therein as well as suitable packaging for the same, which are especially applicable to mammals, e. g. humans, which suffer from or in the future may suffer from neoplasia, e. g. neoplastic disease. In a preferred embodiment of the invention, the subject is a human at increased risk of neoplasia as defined above. It is particularly preferred if the subject is a human suffering from neoplasia as defined above.

In accordance with the present invention, treating or preventing neoplasia, e. g. neoplastic disease, in a mammal in need of such treatment, comprises administering to said mammal an amount therapeutically effective for treating or preventing neoplasia, of one or more than one compound of Formula (I), a physiologically acceptable salt or derivative thereof.

The phrase "therapeutically effective" is intended to describe the amount of each agent which will achieve the goal of improvement in disease severity and the frequency of incidence over treatment of each agent by itself, while avoiding adverse side effects typically associated with alternative therapies.

Said treatment or prevention especially comprises treatment or prevention of neoplastic disease.

The expression "treating or preventing" as used herein with reference to the administration of the compounds of the present invention, is intended to refer to both the therapeutic objective of said administration as well as the therapeutic results actually achieved by said administration. As discussed above, the extent of therapy accomplished by administration of said compounds may range from an amelioration to a significant diminishing of the course of the disease, and beyond to active treatment of the disease, including a reversal of the disease process.

The compounds of Formula (I), physiologically acceptable salts or derivatives thereof, of the present invention may also be combined with other therapeutically active ingredients which would be readily apparent to the skilled artisan in this field, and which will usually be determined by the circumstances under which the therapeutic agent of the present invention is administered. Examples of such other therapeutically active ingredients include, but are not limited to the above agents. However, according to particular embodiment of the present invention, ML3000 is used alone or in combination with one or more cytostatic agents, preferably selected from the cytostatic agents as defined above.

Accordingly, this type of medication provides a means for effectively treating conditions of neoplasia, e. g. neoplastic disease.

In accordance with a regimen which would be used according to the invention, it is contemplated that the compounds of Formula (I), physiologically acceptable salts or derivatives thereof would be administered in combination with other medications used on a regularly scheduled basis. It is also envisioned that administration in combinations could assume a number of different forms and still be within the scope of the present invention. E. g., the compounds of Formula (I), physiologically acceptable salts or derivatives thereof might simply be formulated with one or more of the other therapeutic agents which are to form the intended combination, into a convenient dosage form, such as an oral tablet, containing all of the drugs forming the combination. Varying half-lives for the different drugs could be accommodated by the person skilled in preparing formulations by creating controlled-release forms of said drugs with different release times so that relatively uniform dosing was achieved, or by designing a time-adjusted formulation sequence in which different formulations with suitably varied dosages of the individual compounds are combined for scheduled administration, e. g. a formulation sequence which comprises distinct formulations for hourly, twice-a-day and daily administration. A medicated feed used as the dosage form could also be prepared in accordance with well-known principles in the art of formulation, in which the drugs used in the combination were simply present together in admixture in the feed composition. The present invention also contemplates co-administration in which the combination of drugs is achieved by the simultaneous administration of the drugs to be given in combination. Such co-administration could even be by means of different dosage forms and routes of administration. The present invention further contemplates the use of such combinations in accordance with different but regular and continuous dosing schedules whereby desired plasma levels of the drugs involved were maintained in the mammal being treated, even though the individual drugs making up the combination were not being administered to said mammal simultaneously. All such combinations would be well within the skill of the artisan to devise and administer.

When the compounds of Formula (I), physiologically acceptable salts or derivatives thereof are to be used as active ingredients in the uses, methods and compositions of the present invention, they can be incorporated into standard pharmaceutical dosage forms, which the skilled artisan is familiar with. Basically, any pharmaceutical dosage form may be used in the invention.

In a particular embodiment of the invention, the formulation to be used is a formulation for targeted drug delivery, e. g. a formulation that increases the effective concentration of the drug in the tissue that has given rise to the neoplastic cell, or more specifically in or around the neoplastic cell itself. Examples for such targeted drug delivery systems comprise conjugates, liposomes, micelles and nanoparticulate structures, as known in the art.

Thus, the present invention also relates to pharmaceutical compositions comprising a pharmaceutically acceptable carrier in addition to at least an amount therapeutically effective for stimulating apoptosis, of a compound of Formula (I), a physiologically acceptable salt or derivative thereof as above-defined. Such carriers are known in the art.

E. g., they are useful when administered in systemic or local, oral or parenteral applications and for this purpose are combined with the usual pharmaceutical excipients, diluents and adjuvants, e.g., organic and inorganic inert carrier materials such as water, gelatine, lactose, starch, magnesium stearate, talc, vegetable oils, gums, polyalkylene glycols, etc. These pharmaceutical preparations can be employed in a solid form, e.g., as tablets, capsules, and especially in combination with or for admixture with a palatable food item suitable for mammals; or they can be administered in liquid form, e.g., as solutions and elixirs.

Further pharmaceutical excipients and adjuvants which may be added include preservatives, antioxidants, antimicrobial agents and other stabilizers; wetting, emulsifying, and suspending agents, and anti-caking compounds; fragrance and colouring additives; compositions for improving compressibility, or to create a delayed, sustained or controlled release of the active ingredient; and various salts to change the osmotic pressure of the pharmaceutical preparation or to act as buffers. Such excipients and adjuvants are known to the skilled artisan. Particular dosage forms which have been used with success include a 5% mixed-micelle solution of ML3000 for intravenous injection, a 3% palatable paste, and oral tablets.

The therapeutically effective amount of a compound of Formula (I), a physiologically acceptable salt or derivative thereof, as defined may be administered systemically to said mammal, wherein said systemic administration comprises: (1) injection or infusion into suitable body tissues or cavities of a pharmaceutical composition containing said compound in suitable liquid form such as aqueous solutions, emulsions or suspensions for intraarterial, intra- or transdermal (including subcutaneous), or intraspinal, especially intrathecal, and most commonly for intramuscular or intravenous delivery thereof; or for serving as a depot for delivery thereof; (2) instillation into suitable body tissues or cavities of a pharmaceutical composition containing said compound in suitable solid form, e.g., comprising a matrix of bio-compatible and bioerodible materials in which particles of a solid antitumour compound of Formula (I), a physiologically acceptable salt or derivative thereof, are dispersed, or in which, possibly, globules or isolated cells of a liquid antitumour compound of Formula (I), a physiologically acceptable salt or derivative thereof, are entrapped, for serving as a solid implant composition for delayed-, sustained-, and/or controlled-release delivery thereof; or (3) ingestion or administration of a pharmaceutical composition containing said compound in suitable solid or liquid form for transdermal delivery thereof, for instance a transdermal patch or a subepidermal (subcuticular) implant, for peroral delivery thereof.

A substantial number of the dosage forms described herein may be formulated so as to provide controlled-, sustained-, and/or delayed release of the active ingredient from said dosage form.

A useful controlled release dosage form of ML3000 in accordance with the present invention is one which maintains a ML3000 plasma level greater than 100 ng/mL for most of the day after a single oral dose at 5 mg/kg. Preferred oral controlled release dosage forms of ML3000 in accordance with the present invention are such as maintain a plasma ML3000 concentration greater than 100 ng/mL for a period of time greater than that for which an immediate release dosage form of ML3000 maintains a comparable plasma level, when said immediate release dosage form and controlled release dosage form are administered at the same dose.

Immediate release ML3000 dosage forms containing doses of 2.5 and 5 mg/kg maintain a plasma ML3000 concentration above 100 and 200 ng/mL for 8 hours, respectively.

Preferred peroral dosage forms for systemic administration are solids, e.g., palatable oral compositions such as fast dissolving palatable wafers, tablets, capsules, caplets, etc., and liquids, e.g., solutions, suspensions, emulsions, etc. Pharmaceutical compositions of special types suitable for oral administration to mammals may be used, and include, but are not limited to such items as an oral paste to be delivered to the back of the tongue of the mammal being treated, a granular form to be delivered through incorporation in the mammal's food, and a chewable form wherein the active ingredient is consumed along with the palatable chew, or a chewable form which may deliver the active ingredient by leaching from the body of the chew which is not consumed, during mastication by the mammal being treated.

Said therapeutically effective amount of a compound of Formula (I), a physiologically acceptable salt or derivative thereof as defined may also be administered locally to said mammal, wherein said local administration comprises: (1) injection or infusion into a local site of neoplasia, e. g. neoplastic disease of a pharmaceutical composition containing said compound of formula (I), physiologically acceptable salt or derivative thereof in suitable liquid form for delivery thereof, including components which provide delayed-release, controlled-release, and/or sustained-release of said compound into said local site; or for serving as a depot for delivery thereof wherein said composition provides storage of said compound and thereafter delayed-, sustained-, and/or controlled-release thereof; or (2) instillation of a pharmaceutical composition containing said compound in suitable solid form for serving as a solid implant for delivery thereof, said composition optionally providing delayed-, sustained-, and/or controlled-release of said compound to said local site.

Injections may also be made of pharmaceutical compositions containing the antitumour compound of Formula (I), a physiologically acceptable salt or derivative thereof, where the pharmaceutical composition is in delayed-release, controlled-release, or sustained-release form. These formulations of recognized composition may be solids, semi-solids, gels or other liquid/solid combinations in which an erodible matrix or series of coatings is used to provide a continuous release of the compound of Formula (I), the physiologically acceptable salt or derivative thereof at a predetermined rate or at variable rates if desired. The terms "extended-release" and "long-acting" as well as others are used to describe these formulations. All of these employ various combinations of bioerodible polymers, e.g., various cellulosic polymers, and natural materials, e.g., corn starch and magnesium stearate, to obtain slow and/or uniform dispensing of the compound of Formula (I), a physiologically acceptable salt or derivative thereof contained within the matrix.

In a particular embodiment of the invention, the pharmaceutical composition may comprise a drug targeting system for delivering the drug or drugs preferably to particular cells and/or tissues. Suitable targeting systems have been described in the art.

The therapeutically effective amount for treating or preventing diseases related to neoplasia, e. g. neoplastic disease, of the compound of Formula (I), a physiologically acceptable salt or derivative thereof, is administered to a mammal being treated in an amount expressed as milligrams per kilogram of body weight of said mammal, per day: "mg/kg/day". The expression "per day" as used herein should not be interpreted as necessarily requiring that any particular dosage form be administered on a daily basis to the mammal being treated. The expression "per day" is merely an indication of the smallest convenient but arbitrary segment of time which is being used as part of the overall unit for measuring the dose of effective compound being administered. Depending on the route of application and other details, the daily dosage may be split into a number of sub-doses for subsequent administration in regular intervals, or, when using sustained or controlled release, several daily dosages may be joined into a single depot dosage.

The dose, *i.e.*, the therapeutically effective amount of a compound of Formula (I), a physiologically acceptable salt or derivative thereof for treating or preventing diseases related to neoplasia will usually range from about 0.1 mg/kg/day to about 20.0 mg/kg/day, preferably from about 0.1 mg/kg/day to about 12.0 mg/kg/day, more preferably from about 0.5 mg/kg/day to about 10.0 mg/kg/day, and most preferably from about 0.5 mg/kg/day to about 8.0 mg/kg/day. Typical dosage forms and amounts for ML3000 would include oral administration of ML3000 at a dose rate of 2.5 - 5.0 mg/kg/day of body weight. Particularities of absorption, metabolism and excretion characteristic for the respective tumour type, or found in an individual subject, will have to be taken into account. E. g., special requirements may be needed for patients having FAP, such as a need for higher doses than the average patient.

It is necessary for the skilled artisan not only to determine the preferred route of administration and the corresponding dosage form and amount, but said artisan must also determine the dosing regimen, *i.e.,* the frequency of dosing. In general terms it is most likely that the choice will be between once-a-day (*s.i.d.*) dosing and twice-a-day (*b.i.d.*) dosing, and that the former will provide more rapid and profound therapy, while the latter will provide less profound but more sustained therapy. However, this generalization does not take into account such important variables as the specific type of disease involved, the specific therapeutic agent involved and its pharmacokinetics, and the specific patient (mammal) involved. For an approved product in the marketplace, much of this information is already provided by the results of clinical studies carried out to obtain such approval. In other cases, such information may be obtained in a straightforward manner in accordance with the teachings and guidelines contained in the instant specification taken in light of the knowledge and skill of the artisan. The results which are obtained can also be correlated with data from corresponding evaluations of an approved product in the same assays.

It is also contemplated that in accordance with the present invention there will also be provided a package suitable for use in commerce for treating or preventing conditions of neoplasia, in particular neoplastic disease, in a mammal in need of such treatment, comprising a suitable outer carton and an inner container removably housed therein; enclosed in said container a suitable dosage form of a compound of Formula (I), a physiologically acceptable salt or derivative thereof as described hereinabove; and associated with said carton or container printed instructional and informational material, which may be attached to said carton or to said container enclosed in said carton, or displayed as an integral part of said carton or container, said instructional and informational material stating in words which convey to a reader thereof that said active ingredient, when administered to a mammal in a condition of neoplasia such as neoplastic disease, will ameliorate, diminish, actively treat, reverse or prevent the condition. In a preferred embodiment said package comprising carton and container as above-described will conform to all regulatory requirements relating to the sale and use of drugs for the treatment of animals, including especially said instructional and informational material.

It is also contemplated that in accordance with the present invention there will further be provided a package of the type described immediately above, comprising a suitable container as described; enclosed in said container an oral dosage form of a compound of Formula (I), a physiologically acceptable salt or derivative thereof; and associated with said container printed instructional and informational material as above-described.

The method of the present invention can be further defined to comprise two basic steps: (I) establishing, basically by means known to those skilled in the art, the status of a candidate mammal as presently or prospectively being in a condition of neoplasia such as neoplastic disease, thereby confirming that said mammal is in need of such treatment; and thereupon (II) treating or preventing said condition by administering to said mammal an amount therapeutically effective for treating or preventing said disease related to neoplasia, e. g. neoplastic disease, of an apoptosis-stimulating compound of Formula (I), a physiologically acceptable salt or derivative thereof. The various aspects of Step (II) have already been discussed above in detail. Accordingly, the aspects of Step (I) will now be discussed in detail.

As far as diagnosis is concerned, it is expedient to establish the status of a mammal which is a candidate for treatment in accordance with the present invention as to whether or not the mammal is presently or prospectively in a condition of neoplasia such as neoplastic disease. The expression "presently or prospectively" as used herein is intended to mean that in accordance with the below-discussed methods of making that determination, it is possible to identify a candidate mammal as either being presently in need of such treatment, or as very likely or expected to be in need of such treatment in the short term future. Prospective need of treatment may be established by those determinations of positive factors which from the experience of the artisan lead directly to the condition of disease related to neoplasia. E. g., the artisan may establish from clinical examination of a mammal that it has a condition of neoplasia such as neoplastic disease, and may confirm this conclusion with further evidence from which it may be determined in accordance with established methods of measurement that the mammal will develop a disease related to neoplasia-within the short term future. In human subjects, established risk factors such as smoking, occupational exposure to mutagens, familial occurrence of neoplastic disease etc. may also be considered.

The status of said mammal as presently or prospectively being in said condition of neoplasia such as neoplastic disease, and thus in need of such treatment, is in particular determined by positive results from the clinical examination and evaluation of the respective tissues of the candidate mammal, e.g. by non-invasive procedures including fiberoptic endoscopy, magnetic resonance imaging (MRI) and radiographic methods such as double-contrast barium x-ray. Other clinical symptomology and signs would include those gained from direct examination of the condition of the candidate mammal, e. g. by means of biopsy.

For diagnostic purposes, it may be found expedient to perform in vitro testing, e. g. for determining the sensitivity of an individual neoplastic growth to a treatment with a compound of Formula (I), a physiologically acceptable salt or derivative thereof or composition containing the same, as defined above. For example, a sample obtained by biopsy may be subjected to various concentrations of the compounds and/or compositions of the invention and assayed for reaction. In vitro, apoptosis can be measured by any of a number of methods which the skilled artisan is familiar with. The data thus obtained may serve as a basis for rationally determining dosage and route of application to be used. Thus, the invention generally relates to a method for stimulating apoptosis in a mammalian cell, comprising the use of one or more than one compound of Formula (I), a physiologically acceptable salt or derivative thereof as defined above.

The skilled artisan will appreciate that treatment according to the present invention may also be combined with any suitable non-pharmacological treatment.

### BRIEF DESCRIPTION OF THE DRAWINGS

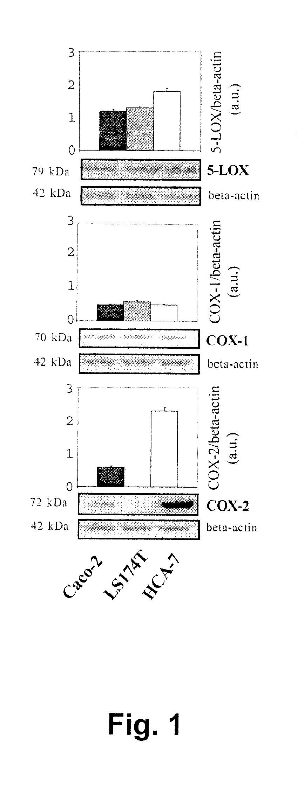

- Fig. 1: is a combination of three Western Blots for individual arachidonic acid metabolism proteins (oxygenases) and their respective densitometric evaluation. In each Western Blot, data were normalized for the housekeeping gene product β-actin. Data are presented in a bar chart in terms of oxygenase protein signal : β-actin signal ratios. Upper row, 5-LOX; middle row, COX-1; lower row, COX-2. In each blot, protein extracts from the following cell types were compared: left column, CACO-2; middle column, LS174T; right column, HCA-7.

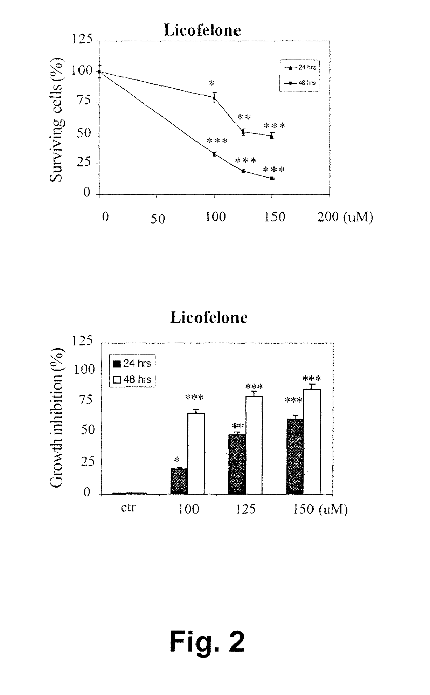

- Fig. 2: is a combination of two charts displaying the dose-effect relationship of licofelone treatment on HCA-7 cell survival/proliferation after 24 and 48 hours, respectively. Upper panel: Survival rate of HCA-7 cells after treatment with increasing doses licofelone; lower panel: relative percentage of growth inhibition. Ctr = control (no licofelone added). Asterisks indicate degrees of significance, with * indicating p< 0.01 (significant), ** indicating p < 0.001 (very significant) and *** indicating p < 0.0001 (extremely significant).

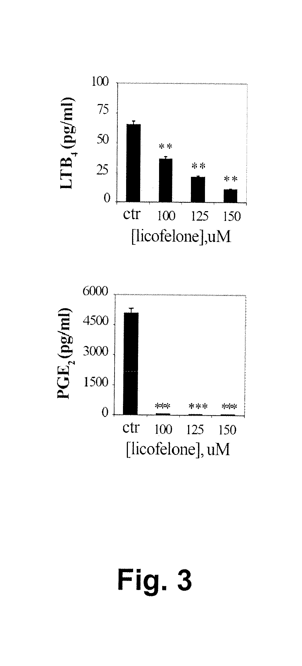

- Fig. 3: is a bar chart displaying the dose-effect relationship of licofelone treatment on LTB₄ and PGE₂ synthesis by HCA-7 cells. Upper panel, LTB₄ production; lower panel, PGE₂ production. Asterisks indicate degrees of significance, with ** indicating p < 0.001 (very significant) and *** indicating p < 0.0001 (extremely significant) .

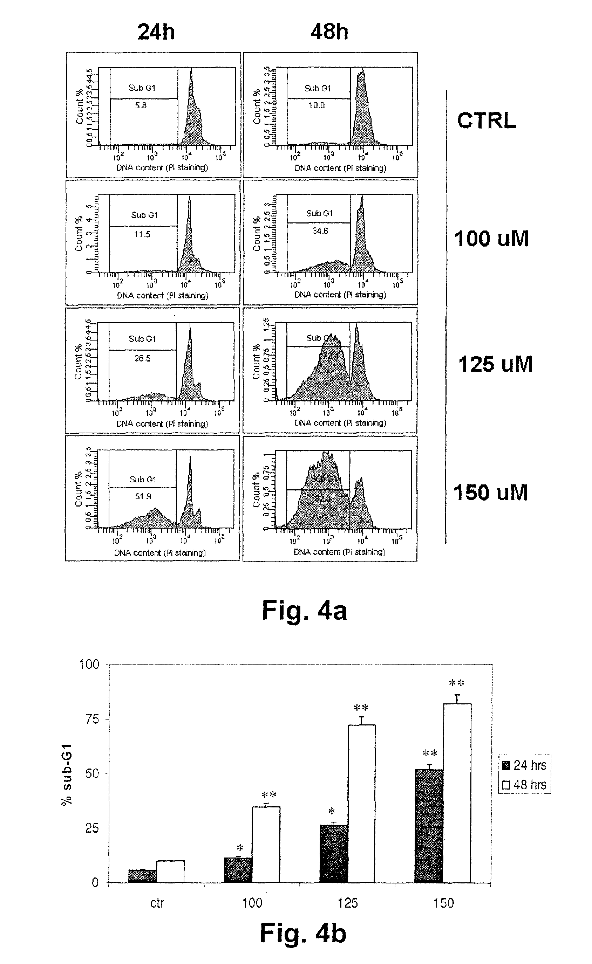

- Fig. 4: shows the dose-effect relationship of licofelone treatment on HCA-7 cell cycle. A, Flow cytometric analysis after staining with propidium iodide (PI) for cellular DNA content. B, percentage of sub-diploid (sub-G1 phase) cells at 24 and 48 hours, respectively, as obtained in A. Ctr = control (no licofelone added). Asterisks indicate degrees of significance, with * indicating p < 0,01 (significant) and ** indicating p < 0,001 (very significant).

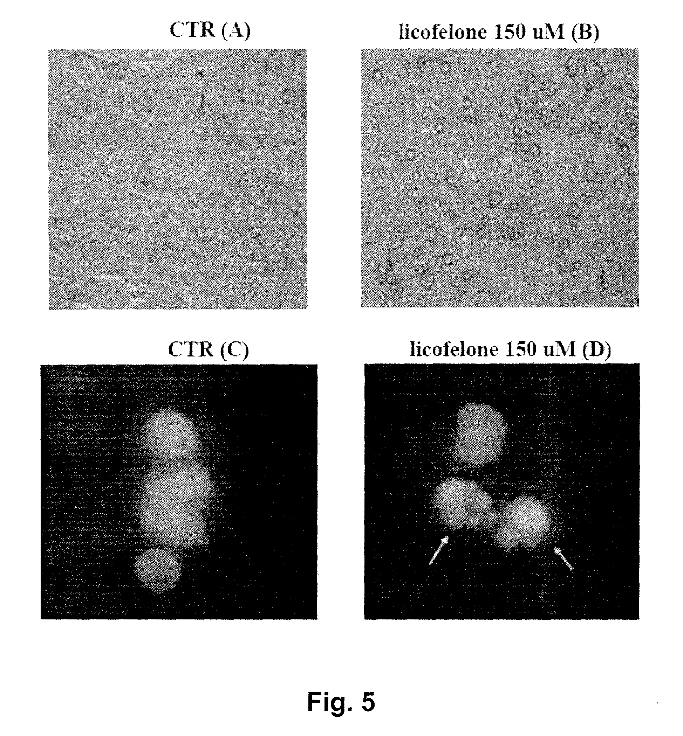

- Fig. 5: shows the effects of licofelone treatment on HCA-7 cell morphology, as seen under light microscope. A and B, phase-contrast microscopy; C and D, fluorescence microscopy after Hoechst 33342 staining to reveal typical apoptotic features of cell nuclei. A and C show control cells (no licofelone added), whereas B and D cells after treatment with licofelone 150 µM. In B, the arrows indicate sites of membrane blebbing; in D, nuclear fragmentation.

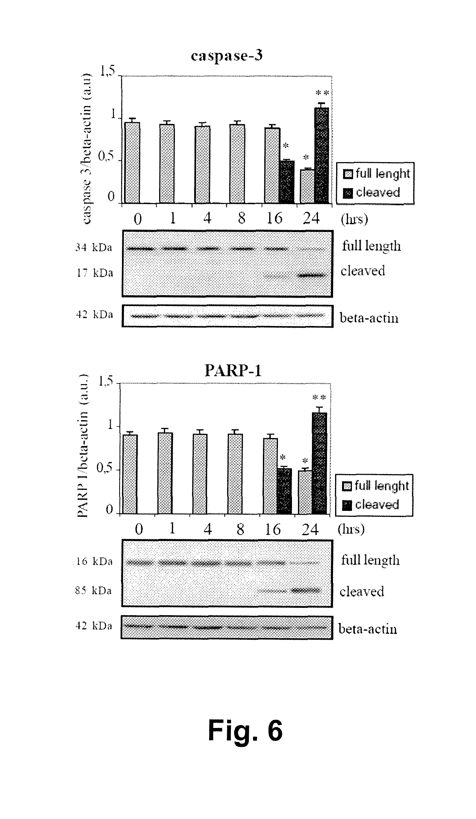

- Fig. 6: is a combination of Western Blots and their respective densitometric evaluations showing total content of the full-length (precursor) and cleaved (activated) forms of the apoptosis-related proteins, caspase-3 and PARP-1, in HCA-7 cells after 0, 1, 4, 8, 16 and 24 hours treatment with licofelone 150 µM. In each Western Blot, data were normalized for the housekeeping gene product β-actin. Asterisks indicate degrees of significance, with * indicating p < 0.01 (significant) and ** indicating p < 0,001 (very significant).

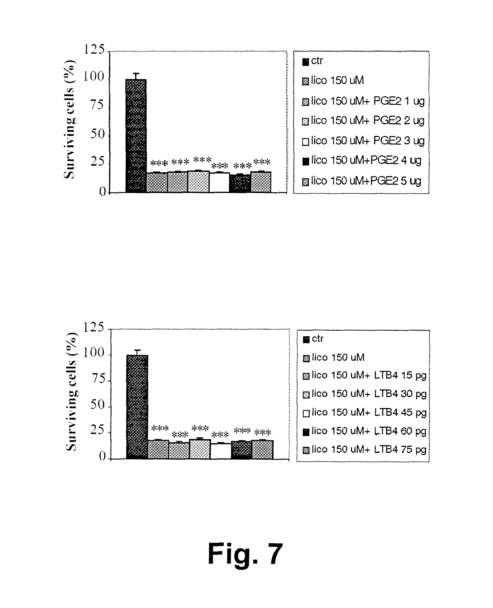

- Fig. 7: shows the effect of exogenous addition of PGE₂ and LTB₄ on HCA-7 survival/proliferation, after 48 hours of treatment in presence of licofelone 150 µM. Upper row, PGE₂ addition; lower row, LTB₄ addition. Asterisks indicate degrees of significance, with *** indicating p < 0.0001 (extremely significant).

### DESCRIPTION OF PREFERRED EMBODIMENTS

In order to further demonstrate the methods and compositions of the present invention, there is presented in the paragraphs which follow specific descriptive examples of typical procedures which may be employed in carrying out said methods. However, said examples are intended to be illustrative only and should not be taken as in any way a limitation of the present invention, for which purpose the present claims are appended hereto.

### EXAMPLE 1

The effects of licofelone, a compound of the invention, on gene expression and proliferation in various tumour cell lines were investigated. In particular, PGE₂ and LTB₄ levels in colon tumour cells, cell cycling behaviour and apoptosis were determined.

### A. MATERIALS AND METHODS

### A.1. Cell Culture

LS174T and CACO-2 were a generous gift of Prof. F. Dall'Olio (Dept. of Experimental Pathology, University of Bologna, Italy); HCA-7 cells were purchased from European Collection of Cell Cultures. These lines were cultured in Dulbecco's modified Eagle's Medium with 4.5 g/L glucose (DMEM), supplemented with 10% (v/v) heat-inactivated FBS, 2 mM L-glutamine, 100 U/ml penicillin and 100 µg/ml streptomycin. Cells were grown in monolayers, incubated at 37°C in a humidified atmosphere of 95% air and 5% CO₂ and routinely passaged using trypsin-EDTA 0.025%.

### A.2. Treatment with Licofelone

For treatment with licofelone (Merckle GmbH, Ulm, Germany), exponentially growing cells were trypsinized, seeded in regular medium (2 x 10⁴ cells/well in 96-well plates; 1.2 x 10⁵ cells/well in 24-well plates; 6 x 10⁵ cells/well in 6-well plates; 1,5 x 10⁶ cells in 25 cm² flasks) and incubated for 24 h. The medium was then replaced either with control medium or with medium containing 100, 125 or 150 µM licofelone. This drug was dissolved in dimethyl sulfoxide (DMSO) and diluted into the medium to obtain the required final concentration before each experiment. Fresh medium, alone or supplemented with licofelone, was completely replaced every 48 hours. To control for an effect of DMSO, control samples were treated in parallel with an equivalent concentration of this solvent.

### A.3. 3-(4,5-dimethylthiazol-2-yl)-2,5-diphenyl tetrazolium bromide (MTT) reduction assay

The effect of licofelone on HCA-7 survival/proliferation was determined colourimetrically using the MTT assay. MTT is a yellow-coloured tetrazolium salt that is taken up and cleaved only by metabolically active cells, which reduce it to a coloured, water-insoluble formazan salt. The solubilized formazan product can be quantified via absorbance at 570 nm measured using a 96-well-format spectrophotometer and the absorbance correlates directly with cell number. 2 x 10⁴ cells/well were plated in a 100 µl volume in 96-well plates and grown for 24 h in DMEM supplemented with 10% FBS. Complete medium containing the indicated amount of licofelone, alone or in association with exogenous PGE₂ and/or LTB₄), was then replaced to each well. At the indicated times, 10 µl of MTT (5 mg/ml) was added and cells were incubated at 37°C for 4 hours. The tetrazolium crystals were solubilized by the addition of 10% SDS in 0.01 N HCl. After overnight incubation at 37°C, the absorbance was measured at 570 nm using a 96-well spectrophotometric microplate reader. Survival/proliferation rate was calculated as follows (A₅₇₀ treatment/A₅₇₀ control x 100). All determinations were carried out in triplicate.

### A.4. Determination of PGE₂ and LTB₄ Levels in HCA-7 Cells

For determination of PGE₂ and LTB₄ production, HCA-7 were seeded in a 24-well plates at the density of 1.2 x 10⁵ cells/well and grown in complete medium for 24 hours. Cells were then serum-starved overnight before being exposed to increasing doses of licofelone (100 - 150 µM) in serum-free medium for additional 24 hours. The harvested medium was centrifuged at 500 x g for 5 min (4°C) to remove floating cells and the supernatant was collected and assayed following customer instructions. The levels of PGE₂ and LTB₄ were measured by enzyme immunoassay. Results are expressed as picograms/ml of medium. Determinations were carried out on three similarly treated wells of cells and the medium from each experiment was assayed at three different dilutions.

### A.5. Protein Extraction and Western Blotting

For Western blot analysis, cells were seeded in a 25 cm² flask at the density of 1,5 x 10⁶ and grown in complete medium for 24 hours. At the end of incubation cells were scraped from flasks and lysed in a buffer containing 10 mM Tris, 1% SDS, 1 mM Na orthovanadate and a protease inhibitors cocktail. Samples containing 50 µg of protein were subjected to gel electrophoresis in 10% polyacrylamide gels in the presence of SDS. The proteins were then transferred electrophoretically to a Hybond-C nitrocellulose membrane at 300 V for 1 h at 4 °C. The membrane was subsequently immersed in 0.5% Ponceau S in 1% acetic acid to stain the proteins and to confirm that equal amounts of protein were loaded in each lane and transferred efficiently. After incubation overnight in a blocking solution containing 5% bovine skim milk powder in 10 mM phosphate buffered saline (PBS, pH 7.4), the nitrocellulose membrane was incubated for 1 h with primary antibodies: anti COX-1, anti COX-2 and anti 5-LOX were used in HCA-7, Caco-2 and LS174T at a dilution of 1 : 500; anti Caspase-3 and anti PARP-1 were used in HCA-7 at the dilution of 1 : 250. The membrane was then washed with PBS buffer to remove the excess unbound antibodies and incubated for 1 h with HRP-conjugated secondary antibody, at a dilution of 1 : 1000. Detection was performed by using the ECL procedure developed by Amersham. Afterward, the membrane was re-blotted with an anti β-actin antibody for normalization and equal protein loading.

### A.6. Analysis of Cell-cycle Distribution After Licofelone Treatment

The cell-cycle distribution for control and drug-treated cells was determined after 24 and 48 hours of treatment. Cells were seeded at the density of 6 x 10⁵ cells/well onto 6-well plates and allowed to adhere for 24 h. Cells were then incubated with licofelone for 24 and 48 hours and then harvested by trypsinization. At the end of the treatment period, cells were fixed in 100% methanol for 30 min at -20 °C, centrifuged for 10 min at 2000 r.p.m., resuspended in 0.1% Triton-X 100 in PBS containing propidium iodide (40 µg/µl), and RNase (60 µg/µl) and incubated at 4 °C for a minimum of 1 h. Subsequently, DNA content was measured using a flow cytometer and the cell-cycle distribution was calculated using the Phoenix Multicycle for Windows cell-cycle analysis software. A minimum of 10'000 events were measured for each sample.

### A.7. Hoechst 33342 Staining

Morphological changes in the nuclear chromatin of cells undergoing apoptosis were detected by staining with the DNA-binding fluorochrome bis-benzimide stain. Briefly, HCA-7 were seeded in a flat-bottomed 96-well plate at the density of 2 x 10⁴ cells/well and grown for 24 hours in complete medium. Cells where then cultured in the presence or absence of licofelone 150 µM at the indicated times; at the end of incubation, Hoechst 33342 (1 µg/ml) was added to each well for at least 20 min. at 37 °C. HCA-7 cells were then washed with phosphate-buffered saline (PBS), fixed in 4% formaldehyde and observed under a fluorescence microscope.

### A.8. Statistical Analysis

All experiments were carried out in triplicate at least twice and results are expressed as mean or percentage mean ± S.E. Data were analysed by Student's t test and a P value < 0.05 was considered to be statistically significant.

### B.RESULTS

### B.1. Evaluation of COX-1, COX-2 and 5-LOX Protein Expression in HCA-7, CACO-2 and LS174T Colon Cancer Cell Lines

First, the level of protein expression of COX-1, COX-2 and 5-LOX in three available human colon cancer cell lines (HCA-7, CACO-2 ad LS174T) was evaluated. As shown in fig. 1, it was found that, while the level of COX-1 was similar in all the cell lines tested, the highest levels of COX-2 and 5-LOX were observed in HCA-7 cells. B.2. Licofelone Inhibits HCA-7 survival/proliferation

In order to evaluate the effect of licofelone on HCA-7 survival/proliferation, cells were treated with increasing concentrations (0-150 µM) of the drug for 24 and 48 hours. As shown in fig. 2, treatment with licofelone was associated with a statistically very significant time and dose-dependent decrease of HCA-7 growth, with approximately 90% inhibition at the dose 150 µM. In particular, we calculated an IC50 value of 125 ± 6.25 µM and 72 ± 3.6 µM after 24 and 48 hours of treatment with licofelone, respectively. Similar molar concentrations of DMSO had no effect on HCA-7 survival/proliferation (data not shown).

### B.3. Licofelone Decreases PGE₂ and LTB₄ Production in HCA-7 Culture Medium

PGE₂and LTB₄ levels in HCA-7 culture medium treated for 24 hours with increasing doses of licofelone were measured. As shown in fig. 3, treatment with licofelone reduced in a dose-dependent manner LTB₄ levels (up to 85% with respect to untreated cells, at the dose of 150 µM) and strongly inhibited PGE₂ production at all the doses tested.

### B.4. Exogenous PGE₂and LTB₄ Addition Do Not Reverse the Effect of Licofelone on HCA-7 survival/proliferation.

In order to verify whether inhibition of HCA-7 growth by licofelone was due to a decrease of these metabolites in culture medium, exogenous PGE₂and LTB₄, , in a concentration range that replaced the endogenous production, were added for 48 hours, in presence of 150 µM licofelone. As shown in fig. 7, neither PGE₂ or LTB₄ administration was able to reverse the antiproliferative effect of the drug, suggesting that the decrease of PGE₂ and LTB₄ production is not correlated with the inhibition of HCA-7 proliferation induced by treatment with licofelone.

### B.5. Treatment with Licofelone Induces HCA-7 Apoptosis in Vitro

In order to investigate whether HCA-7 growth inhibition by licofelone was due to an induction of apoptosis, flow cytometric analysis was performed to detect a subdiploid peak of DNA, indicative of the DNA fragmentation occurring during the apoptotic process. As shown in fig. 4, treatment with licofelone (0-150 µM) resulted in a markedly time and dose-dependent accumulation of sub-G1 phase cells, with a maximum of 85% after 48 hours of treatment. These results have been supplemented by the observation of the typical morphological features of cells undergoing apoptosis. Light microscope examination showed that the appearance of HCA-7 cells was deeply affected by the treatment with licofelone. Cell surface was irregular due to the presence of numerous blebs, while untreated HCA-7 cells had a smooth surface (fig. 5 A, B). Blebbing, a distinguished feature of apoptotic cells, appeared 10-12 hours after the administration of the drug and preceded the morphological changes observed in the nucleus. Observation under fluorescent light upon Hoechst staining (fig.5 C, D) showed that nuclei of treated cells displayed typical apoptotic features. Some of the cells showed at first a condensed perinuclear rim, while others displayed irregular and grossly heterochromatic masses. Within few hours, these two nuclear morphologies evolved into unfragmented or irregularly fragmented picnotic nuclei, respectively. Morphological changes of nuclei always followed the appearance of surface blebs, not earlier than 16-18 hours after drug addition to the culture.

### B.6. Licofelone Activates Caspase-3 and Induces poly-(ADP-ribose)polymerase (PARP) Cleavage

A family of aspartate-specific cysteinyl proteases (caspases) play a pivotal role in the execution of apoptosis. Accordingly, the effect of licofelone 150 µM on caspase-3 activation was examined by western blot analysis in HCA-7 cells. As shown in fig. 6 A, licofelone treatment resulted in a strong and time-dependent cleavage of the 34-kDa pro-enzyme caspase-3 to its active 17-kDa form, with a maximum after 24 hours of treatment. In a second step, we assessed the degradation of the DNA-repair enzyme PARP, the known substrate of caspase-3. As shown in fig. 6 B, incubation with licofelone 150 µM resulted in marked degradation of PARP, seen as a typical 85-kDa band, which was almost complete at 24 hours of treatment.

### C. DISCUSSION

Apoptosis is a controlled form of cell death that plays an important role in maintaining normal tissue homeostasis. In the case of colon physiology, this process is a normal event to terminate the life cycle of intestinal epithelial cells. When the balance between cell proliferation and cell death is affected, cancer develops, suggesting that agents that are able to induce apoptotic death may play a critical role in cancer prevention/therapy, including colon.

As shown herein, licofelone strongly inhibits HCA-7 growth through the induction of apoptosis. Apoptosis was mediated via activation of the caspase cascade, which represents one of the major regulatory steps in the apoptotic pathway. On this basis, the dual COX/5-LOX inhibitor licofelone possess an antitumoural effect in vitro.

Licofelone inhibited HCA-7 proliferation by a mechanism that is independent from its ability to inhibit PGE₂ and LTB₄ production, as supported by the observation that exogenous addition of these two metabolites did not overcome the effect of the drug.

The failure of exogenously added PGE₂ and LTB₄ to reverse HCA-7 growth inhibition suggests that this growth inhibition is due, as reported for others inhibitors of AA metabolism, to an increase in COX and LOX substrates, rather than to a decrease in these enzymes' products. It has been clearly shown, in fact, that unesterified AA may initiate a critical signal for apoptosis. Therefore the induction of this process by inhibitors of AA metabolism may be assumed to be, without wishing to limit the invention by theory, a consequence of its accumulation, rather than the decrease of its metabolites.

Overexpression of COX-2 and 5-LOX, as seen in many colon and other cancer cells, lowers the level of unesterified AA, thereby removing a pro-apoptotic signal and promoting carcinogenesis. On this basis, the development of specific inhibitors targeting the AA-metabolizing pathways may provide novel approaches to slow down and/or kill transformed cells, while they may be expected, on the basis of the mechanism suggested above, to have little or no cytotoxic effect on normal cells, since their effect is not one of directly inflicting damage on vital cell components in the fashion of more aggressive cytostatics, but rather one of resetting the level of an important cellular parameter to its nominal value.

The skilled artisan will be aware that apoptosis is a widely used restrictive mechanism, with the general picture now emerging that cells which are coerced into proliferation by forceful stimuli when they should not be in a proliferative stage will generally undergo apoptosis in order to save the organism, unless their apoptosis is blocked by some other effect. It is therefore to be expected that restoration of normal apoptosis capacity in cells which are running wild, will show an inclination to "understand, repent and die honourably", in particular where uncontrolled growth has already lead to a situation of biochemical stress, such as local scarcity of nutrients in aggressively growing tumours before the establishment of neovascularisation. Such stress situations are known to be strongly pro-apoptotic stimuli.

Moreover, simultaneous inhibition of COX-2 and 5-LOX prevents the shunt of AA toward 5-LOX when COX-2 is blocked, thus reducing the well known side effects otherwise caused by an increase in LOX products, which is considered to be particularly advantageous in situations where the tumour is associated with actual or potential conditions of inflammation and/or microbial infection, as it happens most frequently in the case of epithelial cancer, in particular gastrointestinal cancer.

Thus licofelone and similar compound of formula (I), physiologically acceptable salts or derivatives thereof as defined herein represent an attractive alternative for the treatment of many human neoplasia, used as single therapeutics or in association with other chemotherapeutic agents.

### EXAMPLE 2

A double-blinded, placebo-controlled phase IIb trial is proposed to assess the efficacy of licofelone, a compound of the invention, in patients with bronchial epithelial dysplasia.

The study is designed to test the usefulness of licofelone in the prevention of tumourigenic progression in human subjects at increased risk.

### A. TRIAL DESIGN

- Eligibility for screening: age ≥18 years; ≥30 pack year smoking history; ≥10 year smoking history duration
- Screening evaluation: History; induced sputum
- Prebronchoscopy evaluation (if sputum cytology shows mild-severe dysplasia): History and physical exam; bloodwork (liver function tests, renal function, electrolytes, CBC with platelets, PT/PTT); resting room air oxygen saturation (SaO₂); pregnancy test if woman of reproductive potential; spirometry.
- Baseline bronchoscopy: Bronchoscopy with targeted biopsies and BAL
- Study enrolment: Randomization and enrolment into active protocol if bronchoscopic biopsy shows bronchial epithelial dysplasia; study medication or placebo for 6 months; visits to assess adherence, drug toxicity at 1, 3, and 6 months
- Primary endpoint: Follow up bronchoscopy with targeted biopsies and BAL at 6 months
- Final follow up: Telephone follow up to assess toxicities at 7 months

### B. STUDY PROTOCOL

### B.1. Objectives

### B.1.1. Primary Objective

To determine the efficacy in reducing dysplasia of active drug versus placebo administered over a six-month period in a randomized design in subjects at increased risk for lung cancer due to bronchial dysplasia. Primary efficacy measures will be:
B.1.1.1. the number of dysplastic sites in the bronchial epithelium
B.1.1.2. the grade of bronchial dysplasia on endobronchial biopsy

### B.1.2. Secondary Objective

To evaluate the effect of active drug versus placebo in modulating several intermediate biomarkers (including markers of proliferation, apoptosis, and one or more molecular markers such as cell cycle regulatory parameters to be determined according to the mechanism of action of the active drug and the interest and expertise of an offeror) in several areas of the bronchial epithelium - histopathologically normal and dysplastic - in this cohort; and to evaluate the correlation between regression of bronchial dysplasia (grade or number/area) with the modulations in intermediate biomarkers that will be examined in bronchial biopsies and bronchoalveolar lavage fluid.

### B.2. Background and Rationale

### B.2.1. Introduction

Lung cancer is the leading cause of cancer deaths in the United States in both men and women. In 2003, it is estimated that 171,900 new cases of lung cancer will be diagnosed and that 157,200 people will die from lung cancer. Prognosis from lung cancer is directly related to the stage of disease at time of diagnosis. Unfortunately, 85% of people will present with either regional metastases (intrathoracic lymph node involvement) or distant metastases, making cure very unlikely. Despite treatment innovations such as combined modality approaches and new chemotherapeutic agents with improved efficacy against lung cancer, the 5 year survival after diagnosis of lung cancer has changed only marginally over the past 25 years (from 12% to 15%).

The causal connection between tobacco use and lung cancer is well established. It is estimated that smoking is responsible for 85% of lung cancers and smoking related malignancies account for one-third of cancer deaths. Although the risk of lung cancer decreases upon smoking cessation in comparison to continued smoking, former smokers remain at elevated risk compared to non-smokers for lengthy periods of time and approximately half of lung cancers are diagnosed in former smokers. Thus, new approaches are needed to reduce the lung cancer public health burden.

### B.2.2. Chemoprevention of Lung Cancer

Cancer prevention is the science directed towards reduction in cancer incidence and mortality through screening, early detection, and modulation of individual risk by intervention. The latter may be achieved through behavioral modification such as changes in dietary and exercise habits or through chemoprevention. Chemoprevention targets individuals at increased risk based on both genetic and environmental factors and aims to interrupt carcinogenesis at the pre-invasive stage through the administration of targeted therapies. In early phase trials, the histologic target is often pre-cancerous tissue such as colonic adenomas and bronchial epithelial dysplasia in the lung.

### B.2.3. Bronchial Dysplasia and Lung Cancer Risk

The understanding of bronchial neoplasia has benefited from clinical access to the disease at multiple points during its evolution, thereby allowing development of a preliminary model describing the sequence of histopathologic and molecular changes occurring during lung carcinogenesis. In the central conducting airways of the lung, carcinogenesis is believed to develop through a series of histologically identifiable lesions beginning with squamous metaplasia, progressing through increasingly severe grades of dysplasia up to carcinoma *in situ,* and culminating in invasive carcinoma defined by invasion through the basement membrane. The sequence of events leading to the development of adenocarcinoma is less well understood, although atypical alveolar hyperplasia is believed to be a putative precursor to adenocarcinoma originating in the peripheral lung.

Neoplastic progression from an "initiating" lesion to invasive bronchopulmonary malignancy appears to be a cumulative process occurring over time and involving multiple lesions. It is therefore likely that the above listed histopathologic alterations are associated with changes in: cellular population dynamics (apoptosis and proliferation); cell-cell and cell-matrix communications (levels of growth factors and growth factor receptors); and nuclear/nucleolar morphology, as has been described for other epithelial neoplasms with better defined "stages" of progression (such as the adenoma to carcinoma sequence in colorectal carcinogenesis). To date, numerous changes in the structure, function, and expression of important cellular proteins have been found to occur during lung carcinogenesis. These include activation of oncogenes (e.g., ras, myc, erb families), losses of tumour suppressor activity/physical loci (e.g., p53; Rb; LOH involving 3p, 9p, 5q), suppression of gene expression (e.g.: through abnormalities of DNA methylation), cell cycle abnormalities (e.g.: cyclins, cdk activity, cdk inhibitors), multiple enzyme alterations (e.g.: GSTs, telomerase activity), signal transduction abnormalities (e.g.: RARs, RXRs) as well as additional alterations that may well impact on the process of carcinogenesis (Shin et al., 1994). Some progress has been made in defining a preferred sequence of the alterations occurring during the neoplastic process. For instance, recent investigations indicate that alterations in 3p and 9p occur early in neoplastic progression. The current model is not exact and warrants further investigation to discern its clinical utility in directing therapeutic options at early steps in the neoplastic process.

Patients with bronchial dysplasia - particularly those with ongoing carcinogenic exposures in the form of continued tobacco use or other environmental exposures - carry an elevated risk for metachronous dysplasia and bronchial carcinoma. The knowledge of the biology of the neoplastic process within the lung allows to predict an individual's risk in a general sense, though the majority of population-based or targeted lung cancer surveillance programs investigated to date are limited in both sensitivity and specificity. For these reasons, no surveillance approach is widely accepted nor practiced as a standard of care, although early detection using spiral CT is currently being evaluated in a variety of studies.

### B.2.4. Surrogate Endpoint Biomarkers

Given the prolonged natural history of epithelial neoplastic development in the population at large, progress in chemo-preventive drug development is substantially hindered by the limited ability to evaluate the impact of promising agents within reasonable periods of time and cost. This problem is not unique to cancer prevention, but is shared by every attempt to prevent, rather than treat an existing disease (e.g., prevention rather than treatment of myocardial infarction). One way to improve the efficiency of chemoprevention trials is by substituting target organ-based surrogate endpoint biomarkers (SEBs) for cancer incidence reduction as earlier, though meaningful, measures of preliminary efficacy. As cancer is a histopathologic disease by definition (though driven by germline and somatic genetic alterations), histopathologic markers closest to the incidence of invasive cancer theoretically hold the greatest predictive value among the range of intermediate efficacy markers currently available for application in cancer prevention studies. Such histopathologic changes include reductions in the number, area, or grade of incident pre-invasive neoplastic lesions (atypia of cytologic specimens and dysplasia of histopathologic specimens). The utility and relevance of these markers are difficult to demonstrate definitively since pre-invasive neoplastic lesions are routinely excised either during diagnosis (e.g.: small bronchial dysplasia found incidentally during bronchoscopy or surgical resection for cancer) or as a matter of the acceptable standards of care when their risk for progression to invasive cancer is beyond the comfort level of most clinicians (e.g., carcinoma in situ, colorectal adenomas, cervical dysplasia, bladder dysplasia, actinic keratoses, etc.). In the case of cervical cancer and colorectal cancer, the efficacy of these approaches has been demonstrated; ablation/excision of cervical dysplasia and polypectomy certainly reduce the subsequent incidence of invasive disease, when assessed in the most rigorous methods consistent with ethical standards.

In addition to histopathologic SEBs, markers which characterize fundamental pathophysiologic alterations occurring relatively early during neoplastic progression offer important mechanistic insights, and knowledge about key pharmacodynamic targets, and also may represent tomorrow's validated efficacy biomarkers. Interruption in the balance of cellular population dynamics with progressive accumulation of cells - i.e., an imbalance of proliferation and cell death - is a fundamental property of early neoplasia. Similarly, alterations in cellular morphology - which form the basis for the pathologist's designation of "atypia" or "dysplasia" - are key early markers of the neoplastic process within cells and tissues (occurring both in observable masses of neoplastic cells and in the fields at risk) and therefore are appropriate for further development as markers of preventive efficacy. The nuclear pleomorphism index - which is characterized by several major features including nuclear area, shape, and texture features (particularly when quantitatively evaluated by computer-assisted image analysis to yield the mean and variance of the three parameters of nuclear area, nuclear shape, and nuclear texture; pleomorphic index is then, the sum of the variances of the three measures) and nucleolar morphometry are particularly important morphologic features. Ideally, key genetic markers - or combinations of these - which predispose to neoplastic progression within fields of normally-appearing cells will be identified and may be used for markers of preventive efficacy (e.g., homozygous APC alterations in the cellular population of the colo-rectum of an individual in the sporadic setting or in a genetically predisposed individual such as a FAP subject). Because these markers offer key mechanistic insights that can be applied to develop interventions, identify risk, or evaluate efficacy, these markers are considered to be fundamental to progress in preventing cancer.

### B.3. Summary of Study Plan

The study is a double-blinded, placebo-controlled phase IIb drug study designed to test the efficacy of a chemopreventive drug for lung cancer prevention. It is anticipated that a total of 120 subjects will be recruited. Subjects can be either former or current cigarette smokers; they must have a minimum of 30 pack year cigarette smoking history and have smoked for at least 10 years. Subjects meeting these eligibility criteria will undergo screening sputum cytology. Only subjects with mild-severe atypia on sputum cytology will be scheduled for bronchoscopy. Subjects with bronchial epithelial dysplasia confirmed by histopathologic evaluation of an endobronchial biopsy will be randomized either to placebo or active drug for 6 months. A follow up bronchoscopy will be performed at 6 months. Efficacy will be determined by a comparison between the active drug arm and the placebo arm of the magnitude of change in the number of dysplastic sites within the visible bronchial epithelium and also of the severity of histopathologic dysplasia on biopsy.

### B.4. Subject Selection

All agent administration and the evaluation of clinical efficacy and safety shall be accomplished within 24 months. Evaluation of laboratory-based SEBs and final report submission shall be accomplished within 30 months. The accrual rate of patients and laboratory throughput should therefore be fast enough to permit this schedule.

### B.4.1. Study Population

The study population will be composed of current and former cigarette smokers (defined as having quit for a minimum of 12 months) who have smoked for a minimum duration of 10 years and have accumulated a minimum of 30 pack years total. Ideally, the study population will reflect the demographics of the catchment area in composition based on gender, race, or ethnicity.

### B.4.2. Inclusion Criteria

• ≥ 18 years old
• ≥ 30 pack year smoking
• ≥ 10 year smoking history duration
• Normal kidney and liver function
• Prior history of stage I or II non-small cell lung cancer or stage I/II head and neck cancer allowed, as long as smoking history criteria are met. Subjects must be at least one year from end of curative and adjuvant treatment with no evidence of recurrence.
• Bronchial epithelial dysplasia confirmed by histopathologic evaluation of an endobronchial biopsy

### B.4.3. Exclusion Criteria

• Hypersensitivity to active drug
• Abnormal liver function tests, defined as AST or ALT > upper limits of normal or a history of liver cirrhosis
• History of gastrointestinal ulceration, bleeding, or perforation within the last two years
• Severe lung disease, defined by FEV1 < 40% of predicted value
• Resting hypoxemia while receiving room air
• Chronic renal insufficiency defined by a serum creatinine > 1.5
• Coronary artery disease
• Pregnancy
• Lactation
• Unwillingness to practice contraception if a woman of reproductive age
• Coagulopathy, defined by a history of abnormal bleeding, platelet count < 100,000, PT or PTT > upper limits of normal
• Use of therapeutic warfarin, ticlopidine, or platelet inhibitors with the exception of baby aspirin
• Concurrent medical conditions that, in the judgment of the principal investigator, may interfere with a patient's ability to complete the study or may put the patient at increased risk if enrolled in the study
• Diagnosis of cancer within the last five years, other than head and neck, lung, non-melanoma skin cancer or cervical carcinoma *in situ.* Patients with cancer in remission must be at least five years from original diagnosis.
• Use of concurrent medications that affect drug metabolism including
• Use of any investigational drugs within the last 3 months

### B.5. Agent Information and Administration

### B.5.1. Licofelone

### B.5.2. Dose Groups and Duration of Exposure

Active Treatment: Licofelone po qd for 6 months

Placebo arm: Placebo tablet, 1 tablet po qd for 6 months

### B.5.3. Administration

Subjects will self administer the drugs by mouth.

### B.5.4. Distribution

B.5.4.1. Randomization: A randomization scheme will be developed by the offeror.

B.5.4.2. Blinding and Unblinding Methods: The offeror will be required to maintain the blind for the duration of the study. The offeror may propose conditions under which the study blind for an individual subject is broken prior to study termination. Usually, such an action would require exigent circumstances.

### B.5.5. Dose Modification

For grade 1 toxicity or less, no dose modification will be planned. For grade 2 toxicity definitely, probably, or possibly related to drug, the dose of the study drug will be reduced by 50%. The reduced dose will be maintained until the adverse event (AE) resolves and then the drug dosage will be increased to the original dose. If the adverse event recurs, the reduced drug dosage will be maintained for the remainder of the study. Drug may also be temporarily discontinued for any grade 1 or 2 toxicity that is unacceptable to the participant or physician. For grade 3 or 4 toxicity definitely, probably, or possibly related to the study drug, drug therapy will be discontinued and the subject will be taken off study.

### B.5.6. Adherence/Compliance

The offeror will be required to monitor drug compliance at each visit by means of study diaries and pill counts. Subjects will be evaluated for the effect of the study drug if ≥ 80% compliance is documented.

### B.5.7. Drug Accountability

The offeror will be required to provide a plan for maintaining an adequate record of drug receipt, dispensation, and final disposition pending completion of the study.

### B.6. Criteria for Evaluation and Endpoint Definition

### B.6.1. Primary Efficacy Endpoints:

1. Change in the total number of dysplastic lesions within the visible bronchial epithelium as assessed by bronchoscopy.
2. Change in the severity of the most advanced dysplastic lesion as assessed by histopathology following endobronchial biopsy.

### B.6.2. Secondary Efficacy Endpoints (examples)

1. Cycloxygenase-2
2. 15-Lipoxygenase-1
3. PPAR-γ
4. Ki-67
5. Cyclins D1 and E
6. TUNEL

### B.7. Clinical Evaluations and Procedures

### B.7.1. Schedule of Events

| *Evaluation*/*Procedures* | *Initial Sputum Screening* | *Prebronchoscopy Evaluation* | *Bronchoscopy Visit* | *Study Initiation Visit (Time 0)* | *1 Mo.* | *3 Mo.* | *6 Mo.* | *Telephone Follow Up* |
|---|---|---|---|---|---|---|---|---|
| Informed consent | X | | | | | | | |
| Randomization | | | | X | | | | |
| On-study form | | | | X | | | | |
| Medical history | X | X | X | X | X | X | X | X |
| Physical exam | | X | X | X | X | X | X | |
| Pregnancy test | | X | | | | | | |
| Height/weight | | X | | X | X | X | X | |
| Vital signs | | X | | X | X | X | X | |
| Room Air SaO₂ | | X | | | | | | |
| Serum chemistry | | X | | | X | X | X | |
| Hematology | | X | | | X | X | X | |
| Urine cotinine level | | | | X | | | X | |
| Spirometry | | X | | | | | | |
| Sputum cytology | X | | | | | | X | |
| Bronchoscopy | | | X | | | | X | |
| Adverse events | | | | | X | X | X | X |
| Concomitant medication | X | X | X | X | X | X | X | |
| Dispense /record study medication | | | | X | X | X | X | |
| Off-study form | | | | | | | | X |
| Compliance count | | | | | X | X | X | |

### B.7.2. Pre-intervention Procedures

All eligible subjects will be asked to sign a consent form at the initial evaluation. They will then undergo an initial screening evaluation including medical history and induced sputum collection. If sputum cytology shows mild or worse atypia and no exclusion criteria are triggered, subjects will undergo physical examination, laboratory studies including serum electrolytes, BUN, creatinine, liver function tests, PT/PTT, CBC with platelets, urine cotinine measurement, pregnancy test if applicable, screening spirometry, and bronchoscopy. Only subjects who complete the bronchoscopy and have bronchial epithelial dysplasia confirmed by endobronchial biopsy will go on-study. Study medication will be dispensed at the time of the initiation visit.

### B.7.3. Evaluations during the Study Intervention

Subjects initiated into the study will have follow-up evaluations at 1, 3, and 6 months. At these visits, a complete history and physical exam will be performed, laboratory studies to monitor for drug toxicity will be performed, compliance with the study medicine will be assessed by review of the study diaries and pill counts, and additional study medicine will be dispensed (1 and 3 month visits only).

### B.7.4. Evaluations at Completion of Study Intervention

At 6 months, an off-study form will be completed on all subjects and they will undergo a repeat bronchoscopy. At this time, all areas that were biopsied at the initial bronchoscopy will be biopsied again, as will be any new suspicious areas.

### B.7.5. Follow-up Evaluations

All subjects will receive a telephone call at 7 months where an interval medical history will be obtained and an assessment for any study medication-related toxicity performed. The results of the bronchoscopy will be communicated, if not previously done.

### B.7.6. Methods and Clinical Procedures

### B.7.6.1. Initiation Visit

The initial screening visit will be scheduled by telephone. Subjects will be asked to undergo spirometry (measurement of FEV1 and FVC) and sputum induction at this visit.

### B.7.6.2. Bronchoscopy

Subjects undergoing bronchoscopy will be required to be NPO for at least 6 hours prior to the procedure. They will be allowed to take any oral prescription medicines as scheduled the day of procedure with small sips of water. Subjects will receive conscious sedation, e.g., combination narcotic and short-acting benzodiazepine, and topical anesthesia for the bronchoscopy. The bronchoscopy shall consist of a survey of the readily visible bronchial epithelium, which consists of the main-stem bronchi, lobar bronchi, and segmental bronchi, under either fluorescent or white light. Areas of bronchial mucosa that appear abnormal by bronchoscopy will be measured in cross-diameter (the first measurement should represent the longest axis) and ultimately, will be biopsied following bronchoalveolar lavage. Bronchoalveolar lavage will be performed in the medial segment of the right middle lobe by instillation of 60 ml aliquots of sterile normal saline and gentle aspiration by syringe. Lavage will be repeated until a total of 240 ml of saline has been instilled. Biopsies will then be taken of any abnormally appearing areas of bronchial epithelium. In addition, biopsies will be taken at six predetermined sites: main carina, the carina of the right upper lobe bronchus at its origin from the right main-stem bronchus, the carina of the right middle lobe bronchus at its origin from the bronchus intermedius, the carina of the mediobasilar segmental bronchus of the right lower lobe at its origin from the right lower lobar bronchus, the carina of left upper lobar bronchus at its origin from the left main-stem bronchus, and the carina of the left anterobasilar segment of the left lower lobe at its origin from the left lower lobar bronchus.

### B.8. Statistical Methods

### B.8.1. Sample Size Calculations

The sample size should be calculated based upon the ability to observe a ≥30% preliminary efficacy of licofelone against bronchopulmonary dysplasia using a two-sided alpha of 0.05 with at least 80% power.

## Claims

1. The use of one or more than one compound of Formula (I): 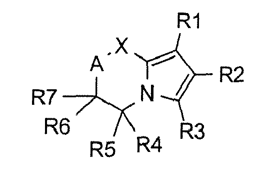 wherein
X represents CR8R9, S, O, NR12 or C(O);
A represents CR10R11 or a bond between X and the atom carrying radicals R6 and R7;
the first of radicals R1, R2, R3 represents
aryl, optionally substituted with one or more than one substituents independently selected among the group consisting of halogen, alkyl, halogenoalkyl, alkoxy, aryloxy, halogenoalkoxy, alkylthio, hydroxy, nitro, alkylsulfinyl, alkylsulfonyl, sulfamoyl, N-alkylsulfamoyl, N,N-dialkylsulfamoyl, alkylsulfonamido and alkylsulfon-N-alkylamido; or
an aromatic or non-aromatic, mono- or bicyclic, optionally benzoannellated, heterocyclic group having 1, 2 or 3 heteroatoms independently selected from N, O and S and optionally being substituted with one or more than one substituents independently selected among the group consisting of halogen, alkyl, halogenoalkyl, alkoxy, aryloxy, halogenoalkoxy, alkylthio, hydroxy, nitro, alkylsulfinyl, alkylsulfonyl, sulfamoyl, N-alkylsulfamoyl, N,N-di-alkylsulfamoyl, alkylsulfonamido and alkylsulfon-N-alkylamido;
the second of radicals R1, R2, R3 represents
alkyl, optionally substituted with one or more than one substituents independently selected among the group consisting of halogen, cycloalkyl, alkoxy, trifluormethoxy, hydroxy and trifluormethyl;
cycloalkyl, optionally substituted with one or more than one substituents independently selected among the group consisting of halogen, alkyl, halogenoalkyl, cycloalkyl, alkoxy, halogenalkoxy and hydroxy; aryl, optionally substituted with one or more than one substituents independently selected among the group consisting of halogen, alkyl, halogenoalkyl, alkoxy, aryloxy, halogenoalkoxy, alkylthio, hydroxy, nitro, alkylsulfinyl, alkylsulfonyl, sulfamoyl, N-alkylsulfamoyl, N,N-dialkylsulfamoyl, alkylsulfonamido and alkylsulfon-N-alkylamido; or an aromatic or non-aromatic, mono- or bicyclic, optionally benzoannellated, heterocyclic group having 1, 2 or 3, heteroatoms independently selected from N, O and S and optionally being substituted with one or more than one substituents independently selected among the group consisting of halogen, alkyl, halogenoalkyl, alkoxy, aryloxy, halogenoalkoxy, alkylthio, hydroxy, nitro, alkylsulfinyl, alkylsulfonyl, sulfamoyl, N-alkylsulfamoyl, N,N-di-alkylsulfamoyl, alkylsulfonamido and alkylsulfon-N-alkylamido;
the third of radicals R1, R2, R3 represents
H, alkyl, halogenoalkyl, hydroxyalkyl, -CHO, -COOH, halogen, cyano, alkylsulfonyl, sulfamoyl or A'-Y;
wherein
A' represents alkylene or alkenylene, optionally substituted with hydroxy or alkoxy;
Y represents -COOH, SO₃H, OPO(OH)₂, OP(OH)₂, -CHO or tetrazolyl;
or the second and the third of radicals R1, R2, R3 represent,
together with the atom they are attached to, saturated or unsaturated cycloalkyl;
R4-R11, which may be the same or different, represent
hydrogen, alkyl, hydroxyalkyl, alkoxyalkyl, hydroxy, COOH or acyloxy, where vicinal radicals may also represent bonds or geminal radicals, together with the C atom they are attached to, may also represent carbonyl or cycloalkyl;
R12 represents hydrogen, alkyl or phenyl,
and physiologically acceptable salts and derivatives thereof,
for the prevention and/or treatment of neoplasia.

2. The use according to Claim 1, wherein the first and the second of radicals R1, R2, R3 independently represent an optionally substituted aryl or aromatic heterocyclic residue.

3. The use according to Claim 1 or Claim 2, wherein the third of radicals R1, R2, R3 represents COOH or A'-Y, wherein Y is COOH and A' represents alkylene.

4. The use according to Claim 1 wherein said compound of formula (I) is [6-(4-chlorophenyl)-2,2-dimethyl-7-phenyl-2,3-dihydro-1 H-pyrrolizine-5-yl]-acetic acid of the formula (la) 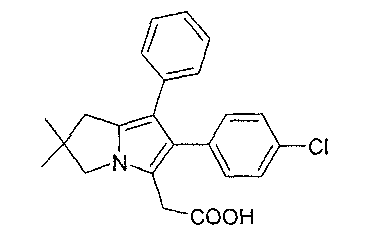 a physiologically acceptable salt or a physiologically hydrolysable ester thereof.

5. The use according to any one of Claims 1 to 4, where the neoplasia is selected from the group consisting of papilloma, carcinoma, adenoma and adenocarcinoma.

6. The use according to any one of Claims 1 to 5, where the neoplasia is a fast-growing malignancy.

7. The use according to any one of Claims 1 to 6, where the neoplasia is a human airway carcinoma.

8. The use according to any one of Claims 1 to 6, where the neoplasia is a human colorectal carcinoma.

9. The use according to any one of Claims 1 to 6 or 8, where the tumor is a tumor of adenomatous polyposis.

10. The use according to claim 9, wherein the adenomatous polyposis is familial adenomatous polyposis.

11. The use according to any of Claims 1 to 10, where the neoplasia comprises a neoplastic cell which is multi-drug resistant (MDR).

12. The use according to any of Claims 1 to 11, where the neoplasia comprises a neoplastic cell which is negative for COX-2.

13. The use according to any of Claims 1 to 12, where the treatment comprises further stimulation of cell death by a method selected from irradiation, heating and treatment with a cytostatic agent.

14. The use according to any one of Claims 1 to 13 for the stimulation of apoptosis.

15. The use according to Claim 14 for restoring the ability to undergo apoptosis in a cell having lost the same.

16. Pharmaceutical composition, comprising one or more than one compound of Formula (I) as defined in any one of Claims 1 to 4, or a physiologically acceptable salt or derivative thereof, and one or more cytostatic agents selected from the group consisting of alkylating agents, antimetabolites, plant alkaloids and other natural products, cytotoxic antibiotics and related substances, platinum compounds, methylhydrazines, monoclonal antibodies, sensitizers used in photodynamic and/or radiation therapy, receptor inhibitors, further antineoplastic agents, and combinations thereof.

17. A method of treating or preventing neoplasia in a subject in need thereof, comprising administration to said subject of one or more than one compound of Formula (I) as defined in any one of Claims 1 to 4, or a physiologically acceptable salt or derivative thereof.
